# EUROPEAN PATENT SPECIFICATION

(11) **EP 3 236 937 B1**
(45) Date of publication and mention of the grant of the patent: **04.05.2022**
(21) Application number: 15820533.6
(22) Date of filing: 23.12.2015
(51) Int. Cl.: A61K 9/00, A61K 38/09, A61K 38/31, A61K 47/10, A61K 47/24, A61K 47/26, A61K 9/127, A61K 38/095, A61P 5/02

(54) **CONTROLLED-RELEASE FORMULATIONS**
RETARD-FORMULIERUNGEN
FORMULATIONS À LIBÉRATION CONTRÔLÉE

(30) Priority: 23.12.2014 GB 201423134
(43) Date of publication of application: 01.11.2017
(73) Proprietor: Camurus AB, 223 70 Lund (SE)
(72) Inventor: TIBERG, Fredrik, 223 70 Lund (SE); JOHNSSON, Markus, 223 70 Lund (SE); BARAUSKAS, Justas, 223 70 Lund (SE)
(74) Representative: Dehns
(86) International application number: PCT/EP2015/081191
(87) International publication number: WO 2016/102683

(56) References cited:
- WO-A1-2005/117830
- WO-A1-2013/032207
- WO-A1-2013/144289
- KI MIN-HYO ET AL: "A new injectable liquid crystal system for one month delivery of leuprolide", JOURNAL OF CONTROLLED RELEASE, vol. 185, 29 April 2014 (2014-04-29), pages 62-70, XP028854118, ISSN: 0168-3659, DOI: 10.1016/J.JCONREL.2014.04.034 cited in the application

## Description

### Field

The present invention relates to formulation precursors (pre-formulations) comprising lipids that upon exposure to water or aqueous media, such as body fluids, spontaneously undergo at least one phase transition, thereby forming a controlled release matrix which optionally is bioadhesive.

### Background

Many bioactive agents including pharmaceuticals, nutrients, vitamins and so forth have a "functional window". That is to say that there is a range of concentrations over which these agents can be observed to provide some biological effect. Where the concentration in the appropriate part of the body (e.g. locally or as demonstrated by serum concentration) falls below a certain level, no beneficial effect can be attributed to the agent. Similarly, there is generally an upper concentration level above which no further benefit is derived by increasing the concentration. In some cases increasing the concentration above a particular level results in undesirable or even dangerous effects.

Some bioactive agents have a long biological half-life and/or a wide functional window and thus may be administered occasionally, maintaining a functional biological concentration over a substantial period of time (e.g. 6 hours to several days). In other cases the rate of clearance is high and/or the functional window is narrow and thus to maintain a biological concentration within this window regular (or even continuous) doses of a small amount are required. This can be particularly difficult where non-oral routes of administration (e.g. parenteral administration) are desirable or necessary, since self-administration may be difficult and thus cause inconvenience and/or poor compliance. In such cases it would be advantageous for a single administration to provide active agent at a therapeutic level over the whole period during which activity is needed.

There is an enormous potential in the use of peptides (including proteins) for treating various disease states, as well as in prophylaxis and in improving general health and well-being of subjects. However, the performance of administered peptide agents is generally limited due to poor bioavailability, which in turn is caused by the rapid degradation of peptides and proteins in biological fluids. This increases the dose which must be administered and in many cases restricts the effective routes of administration. These effects are further exaggerated by the often limited permeability of peptides and proteins across biological membranes.

Peptides and proteins that are administered to the mammalian body (e.g. orally, intramuscularly etc.) are subject to degradation by various proteolytic enzymes and systems present throughout the body. Well known sites of peptidase activity include the stomach (e.g. pepsin), and the intestinal tract (e.g. trypsin, chymotrypsin, and others) but other peptidases (e.g. aminopeptidases, carboxypeptidases, etc.) are found throughout the body. Upon oral administration, gastric and intestinal degradation reduces the amount of peptide or protein which potentially could be absorbed through the intestinal surface lining and thereby decreases their bioavailability. Similarly, free peptides and proteins in the mammalian blood stream are also subject to enzymatic degradation (e.g. by plasma proteases etc.). These factors make peptides one of several categories of bioactive agents for which controlled delivery is potentially a major advantage.

Some patients undergoing treatment will require a therapeutic dose to be maintained for a considerable period and/or ongoing treatment for many months or years. Thus a depot system allowing loading and controlled release of a larger dose over a longer period would offer a considerable advantage over conventional delivery systems. Most established controlled-delivery systems rely on polymers, especially polymers that degrade in the body. These include the Alkermes Medisorb^{®} delivery system consisting of microspheres of biodegradable polymers. Such polymer microsphere formulations must generally be administered by means of a sizable needle, typically of 0.9 mm nominal outer diameter (20-gauge) or wider. This is necessary as a result of the nature of the polymeric dosing systems used, which are typically polymer suspensions.

The poly-lactate, poly-glycolate and poly-lactate-co-glycolate polymers typically used for degrading slow-release formulations are also the cause of some irritation in at least some patients. In particular, these polymers typically contain a certain proportion of acetic acid impurity, which will irritate the injection site on administration. When the polymer then breaks down, lactic acid and glycolic acid are the degradation products so that further irritation is caused. As a result of the combined effects of wide-needle administration and irritant contents, discomfort at the site of administration and the formation of connective scar tissue are greater than desirable.

From a drug delivery point of view, polymer depot compositions generally have the disadvantage of accepting only relatively low drug loads and having a "burst/lag" release profile. The nature of the polymeric matrix, especially when applied as a solution or pre-polymer, causes an initial burst of drug release when the composition is first administered. This is followed by a period of low release, while the degradation of the matrix begins, followed finally by an increase in the release rate to the desired sustained profile. This burst/lag release profile can cause the *in vivo* concentration of active agent to burst above the functional window immediately following administration, and then drop back through the bottom of the functional window during the lag period before reaching a sustained functional concentration for a period of time.

A lipid-based, slow-release composition is described in WO2005/117830. This is a highly effective formulation of two key lipid components and an organic solvent. The formulations of that disclosure provide many advantages over polymer based systems including improved release profile, ease of use, ease of manufacture and/or biocompatibility.

In view of the advantages of the diacyl lipid / phospholipid based system disclosed in WO2005/117830, attempts have been made to modify the system by the introduction of an additional "crystal hardener" component.. One such modified system is disclosed in WO2013/032207. The system of this document comprises a minimum of three components plus a solvent since a "crystal hardener" is required in addition to a sorbitan ester and a phospholipid. Not only does this make the system more complex to formulate and validate for pharmaceutical manufacture but many of the proposed crystal hardeners have their own bioactivity. These include retinyl palmitate, which has been implicated as a possible carcinogen, and (in all examples) tocopherol acetate (vitamin E acetate) which will evidently be bioactive.

Although the system of WO2013/032207 does not currently match the performance, simplicity or injectability of the WO2005/117830 system, it would be an advantage if this system could be simplified, and in particular if it could be made effective in the absence of bioactive agents or other "crystal hardeners".

WO2013/144289 relates to vesicular formulation for the treatment of rosacea. The vesicular formulations comprise one or more phospho or sulpholipids and one or more surfactants.

The present inventors have now established that by providing a pre-formulation according to claim 1 comprising at least one fatty acid ester of a sorbitan comprising a sorbitan head group and one to three fatty acyl tail groups, at least one phospholipid (such as phosphatidyl choline or phosphatidyl ethanolamine), at least one biocompatible, oxygen containing, low viscosity organic solvent in carefully controlled proportions, a pre-formulation may be generated which provides a complementary system to known depot formulations based on the combination of diacylglycerols and phospholipids, without the need for an additional crystal hardener component. By use of specific components in carefully selected ratios a depot formulation can be generated having a combination of properties matching or exceeding the performance of known sorbitan-based lipid controlled-release compositions.

In particular, the pre-formulation shows an acceptable release profile, is easy to manufacture, may be sterile-filtered, has sufficiently low viscosity (allowing administration through a typical needle), allows a high level of bioactive agent to be incorporated (thus potentially allowing a smaller amount of composition and/or active agent to be used), requires shallow injection and/or forms a desired non-lamellar depot composition *in vivo* having a "non-burst" release profile. The compositions can be administered by i.m., or s.c. and are suitable for self-administration.

Advantages of the compositions of the present invention over polymer formulations, such as PLGA spheres, include the ease of manufacture (including sterilization), handling and use properties combined with low initial release ("non-burst profile") of active agent.

### Summary of the invention

Viewed from a first aspect, the invention thus provides a pre-formulation according to claim 1.

Generally, the aqueous fluid will be a body fluid such as fluid from a mucosal surface, tears, sweat, saliva, gastro-intestinal fluid, extra-vascular fluid, extracellular fluid, interstitial fluid or plasma, and the pre-formulation will form a liquid crystalline phase structure when contacted with a body surface, area or cavity (e.g. *in vivo*) upon contact with the aqueous body fluid. The pre-formulation of the invention may optionally contain a certain amount of water prior to administration, but this will not be sufficient to lead to the formation of the necessary liquid crystalline phase prior to administration.

Viewed from a second aspect the invention provides a pharmaceutical formulation comprising the pre-formulation of the first embodiment, which may additionally comprise at least one pharmaceutically tolerable carrier, preservative, excipient or other pharmaceutically tolerable component.

Viewed from a third aspect the invention provides a pre-formulation for use as a medicament according to claim 13.

Viewed from a fourth aspect the invention provides a pre-filled administration device containing a pre-formulation according to the first aspect of the invention.

Viewed from a fifth aspect the invention provides a kit comprising an administration device as hereinbefore defined.

### Detailed description of the Invention

Formulations of the present invention generate a non-lamellar liquid crystalline phase following administration. Formulations of the invention differ from known lipid systems based on glycerol dioleate and phosphatidyl choline (GDO/PC) in that the glycerol-derived diacyl lipid is replaced, largely replaced or at least supplemented with at least one fatty acid ester of a sorbitan comprising a sorbitan head group and one to three fatty acyl tail groups.

WO2013/032207 A1 discloses pre-formulations comprising a fatty acid sorbitan ester, a phospholipid, a liquid crystal hardener and ethanol. The compositions described therein are disclosed as being suitable for slow release of active agents. Further data for the slow-release of leuprolide is given by the same authors in J. Controlled Release 185 (2014), 62-70. In WO2013/032207 the role of the liquid crystal hardener is indicated as being essential to increasing the curvature of the non-lamellar phase. The liquid crystal hardener is disclosed in this publication as a compound being free of an ionizable group, having a hydrophobic moiety of 15 to 40 carbon atoms and having a triacyl group or a carbon ring structure. Specific Examples include triglycerides, retinyl palmitate, tocopherol acetate, cholesterol, benzyl benzoate, and mixtures thereof. The same liquid crystal hardeners are employed in the formulations of WO2014/104784 A1, WO2014/104788 A1 and WO2014/104791 A1. Ubiquinone is additionally suggested as a liquid crystal hardener in these publications.

The presence of a liquid crystal hardener is required in the above-mentioned controlled-release systems based on sorbitan-ester and phospholipid. The clear disclosure of WO2013/032207, WO2014/104784, WO2014/104788 and WO2014/104791 is that sorbitan esters and phospholipids cannot be formulated together in such a way as to produce an effective slow-release lipid formulation, without the addition presence of this liquid crystal hardener.

The present inventors have now established that useful slow-release compositions can be provided by the combination of at least one fatty acid ester of a sorbitan comprising a sorbitan head group and one to three fatty acyl tail groups with at least one phospholipid, in the absence of an additional liquid crystal hardener. Specifically, it has been determined that the presence of a liquid crystal hardener is unnecessary if the ratios of sorbitan ester and phospholipid are carefully controlled. This is entirely unexpected in view of the criticality of this component taught by the previous disclosures of corresponding systems.

The present invention thus provides a substitute to known slow-release formulations based on the sorbitan ester / phospholipid systems having the benefit of not requiring one or more liquid crystal hardeners. It will be appreciated that formulations of the invention are intended for pharmaceutical use and therefore each component of the composition, as well as the composition as a whole, must satisfy stringent health and safety criteria. Essential components of the present invention include a sorbitan ester, phospholipid and a biocompatible, oxygen containing, low viscosity organic solvent. The latter two components are widely used in pharmaceutical preparations. Sorbitan esters are commercially available from various suppliers such as Croda (e.g., Span^{®} 80). All of these components have prior use in pharmaceutical products, thus compositions of the present invention are likely to conform to local pharmaceutical standards and be non-harmful even when administered on a regular basis.

Sorbitan ester/phospholipid systems known in the prior art feature the additional presence of a liquid crystal hardener. Whilst some hardeners suggested by the prior art may be pharmaceutically acceptable, several others are clearly not desirable, especially where a patient is required to take these compositions on a regular basis and may be exposed to relatively large quantities of this component.

The purpose of a liquid crystal hardener is to assist in generating a non-lamellar phase on contact with an aqueous fluid. It is likely in many cases that the suggested and exemplified liquid hardeners could exhibit a physiological effect. It also seems unlikely that regulatory approval would be permitted for several hardeners suggested in the prior disclosures. Obtaining regulatory approval can be an onerous task. The liquid crystal hardener may also be expensive and be detrimental to the stability of any peptide present, particularly where the liquid crystal hardener is tocopherol. These problems are addressed by the present invention in which no liquid crystal hardener is needed.

The present invention provides a complementary system to known slow-release lipid systems based on the combination of diacyl glycerols (DAG) and/or tocopherol with phosphatidyl choline (PC) or phosphatidyl ethanolamine (PE). Whilst it is known that the release properties of the DAG/PC or DAG/PE systems can be tuned to suit the application of interest, i.e. so as to produce a week-long or month-long slow release product, it would clearly be advantageous to provide complementary systems which may be tuned to have different release profiles, for instance those not attainable with the DAG/PC system. The use of a sugar-lipid component (i.e. a sorbitan ester in place of DAG and/or tocopherol) may also allow for different loadings of active agents which may, for instance, be of lower solubility in known DAG/PC systems.

The present invention therefore provides a complementary system to known lipid systems based on the combination of a sorbitan ester with a phospholipid, by carefully selecting the ratio of sugar ester:phospholipid so as to make the presence of an additional liquid crystal hardener component redundant.

Pre-formulations of the invention exclude liquid crystal hardeners. The liquid crystal hardeners disclosed in WO2013/032207 A1 are excluded from pre-formulations of the present invention.

It will be appreciated that it may be difficult or even impossible to exclude components such as certain liquid crystal hardeners completely. These may, in one embodiment applicable to all aspects of the invention, be present at trace amounts in components i) and/or ii). In this context, the term "exclude" relates to a level of component, such as crystal hardener, which is below 1,000 ppm by weight relative to the composition as a whole. Preferably, the level of the excluded crystal hardener is below 500 ppm, more preferably below 300 ppm, still more preferably below 100 ppm. In an alternative embodiment, "exclude" may be taken to exclude to a very high level, such as to less than 1 ppm, less than 0. 1ppm, or even to below the limit of detection.

In one aspect the presence of non-peptide active pharmaceutical ingredients is excluded in pre-formulations of the present invention. In another aspect pre-formulations of the present invention exclude the presence of any peptide or non-peptide active pharmaceutical ingredient entirely.

It is a surprising result that the proportions of components selected can result in a pre-formulation which exhibits lower or similar burst-release as well as similar overall release profiles than some known lipid systems based on sorbitan-ester/phospholipid/liquid crystal hardener when formulated with a peptide active agent. Formulations of the present invention have comparable low burst-release properties to some known formulations based on diacyl glycerols (e.g. glycerol dioleate (GDO)) and phosphatidyl choline (PC).

### Component i) - Sorbitan ester

Component i) of the present invention is at least one sorbitan ester. Such esters comprise a polar sorbitan "head" group, and one to three fatty acyl non-polar "tail" groups. Component i) of the invention may be mono-esters, di-esters, tri-esters, tetra-esters or mixtures thereof. Typically, component i) will comprise at least some di-ester of a sorbitan.

It will be appreciated that various stereoisomers of the head group may exist. The present invention is not limited to any particular stereoisomer of the polar head group. However, the polar head group is sorbitan. Obviously, any sorbitan ester present as component i) must be biotolerable.

Examples of non-polar "tail" groups include C₆-C₃₂ alkyl and alkenyl groups, which are typically present as the esters of long chain carboxylic acids. These are often described by reference to the number of carbon atoms and the number of unsaturations in the carbon chain. Thus, CX:Z indicates a hydrocarbon chain having X carbon atoms and Z unsaturations. C12 to C24 fatty acyl groups are highly suitable, particularly with zero, one, two or three unsaturations in the hydrocarbon chain. C16 to C20 are highly preferred, particularly with 0 to 3 unsaturations. Examples particularly include lauroyl (C12:0), myristoyl (C14:0), palmitoyl (C16:0), phytanoyl (C16:0), palmitoleoyl (C16:1), stearoyl (C18:0), iso-stearoyl (C18:0), oleoyl (C18:1), elaidoyl (C18:1), linoleoyl (C18:2), linolenoyl (C18:3), arachidonoyl (C20:4), behenoyl (C22:0) and lignoceroyl (C24:9) groups. Thus, typical non-polar chains are based on the fatty acids of natural ester lipids, including caproic, caprylic, capric, lauric, myristic, palmitic, phytanic, palmitolic, stearic, iso-stearic, oleic, elaidic, linoleic, linolenic, arachidonic, behenic or lignoceric acids, or the corresponding alcohols. Preferable non-polar chains are palmitic, stearic, iso-stearic, oleic and linoleic acids, particularly oleic acid.

Component i) comprises at least one fatty acid ester of sorbitan. The sorbitan ester comprises a sorbitan head group and one to three fatty acyl tail groups. Such esters may be mono-, di- or tri-esters and component i) may comprise a mixture of two or more such esters. In one embodiment, component i) will comprise a mixture of mono-, di- and tri-fatty acid esters of sorbitan. In all of these esters, the "fatty acid" or "fatty acyl" groups will preferably be the preferred groups referred to herein, such as palmitic, stearic, iso-stearic, oleic and/or linoleic acids.

In one preferred embodiment, component i) will comprise a fatty acid diester of sorbitan. Component i) may comprise at least 20% of such a sorbitan diester by weight, preferably at least 25% and more preferably at least 30%, relative to the total amount of i). In one embodiment, component i) may comprise a fatty acid diester of sorbitan as the largest component, particularly the largest component of a mixture of mono-, di- and tri- fatty acid esters of sorbitan. In all of these esters, the "fatty acid" or "fatty acyl" groups will preferably be the preferred groups referred to herein, such as palmitic, stearic, iso-stearic, oleic and/or linoleic acids.

Di-, tri- and tetra-esters, where present, will preferably comprise a sorbitan head group with an ester group attached to the primary (i.e. C-6) hydroxyl group of the sugar head group, and at least one ester group attached to at least one other hydroxyl group of the head group, preferably to the C-5 hydroxyl group.

It will be appreciated that even essentially pure sorbitan-esters may comprise a fraction of other esters, such that most commercial preparations will be a mixture of mono, di- and tri-ester.

The present inventors have determined that commercially available Span^{®}80 from various suppliers, although referred to and marketed as the mono-oleate, may comprise a significant fraction of the di- tri- and tetra-oleate. This is corroborated by several sources, which are set out in Table 1.

**Table 1. Chemical composition of Span^{®}80 according to various sources**

| **Mixture** | **Source** | | **Mono-esters** | **Di-esters** | **Tri-and tetra-esters** | **Reference** |
|---|---|---|---|---|---|---|
| S1 | Span 80 (Wako Junyaku, Japan) | | 20 mol% | 49 mol% | 31 mol% | Kato et al., Langmuir 2008, 24, 10762-10770 |
| S2 | Span 80 from various sources (probably Croda) | | 52% | 34% | 14% | Garti et al., JAOCS 1983, 60, 1151-1154 |
| S3 | Span 80 from various sources | SMO ex Croda | 15-20% | 35% | 25% | J. L. Humphrey, 2007. PhD Thesis, University of Hull (publically available on the internet) |
| S4 | | SMO | 15% | 40% | 35% | |
| S5 | | Sorbitan oleate | 15% | 35% | 45% | |
| S6 | Span 80 (Sigma-Aldrich) | | 32% | 36% | 26% | M. V. Gonzalez-Rodriguez et al., J. Sep. Sci. 2010, 33, 3595-3603 (citing original works of Wang and Fingas, J. High Resolution Chromatogr. 1994, 17, 15-19; Wang and Fingas, J. High Resolution Chromatogr. 1994, 17, 85-95) |

Where component i) comprises a mixture of different esters, it is preferred that the total amount of mono- and di-esters is at least 40 wt.%, preferably at least 50 wt.% of component i), such as at least 60 wt.% of component i).

It is preferred that component i) comprises at least 10 wt.% of a mono ester of sorbitan relative to the total amount of i) , preferably 20 wt.% or more. In one preferred embodiment, component i) is Span 80, such as at least one of S1 to S6 (of Table 1) or mixtures thereof.

It will be appreciated that reversed lipid phases form spontaneously on contact with an aqueous fluid and therefore the total content by weight of components i) and ii) in the formulation is not critical. More important is the relative proportion and the behaviour of the mixture.. Typically, the lower wt.% limit of component i) in the pre-formulation is 20 wt.%, preferably more than 30 wt.%, most preferably more than 40 wt.%. The upper wt.% limit of component i) in the pre-formulations is generally 80 wt.%, preferably less than 70 wt.%, more preferably below 60 wt.%. Preferred ranges for component i) are thus 20-80 wt.%, preferably 30-70 wt.%, more preferably 40-60 wt.%, such as 45-55 wt.%.

### Component ii) - Phospholipid component

Component "ii)" in lipid matrices of the present invention is at least one phospholipid. In a preferred aspect, the phospholipid comprises at least one phosphatidyl choline (PC) or at least one phosphatidyl ethanolamine (PE) or mixtures thereof and may consist essentially of these components or consist of these. As with component i), this component comprises a polar head group and at least one non-polar tail group. The difference between components i) and ii) lies principally in the polar group. The non-polar portions may thus suitably be derived from the fatty acids or corresponding alcohols considered above for component i). The dominant component in PC or PE will contain two non-polar groups. Again, all of the preferable groups indicated above for component i) apply correspondingly to component ii). In particular, C12 to C24 fatty acyl groups are highly suitable, particularly with zero, one, two or three unsaturations in the hydrocarbon chain. C16 to C20 are highly preferred, particularly with 0 to 3 unsaturations. C18 groups (again saturated or with 1-3 unsaturations) are most preferred and may be combined with any other suitable non-polar group, particularly C16 groups.

Any phospholipid, such as phosphatidyl choline or phosphatidyl ethanolamine portion may be derived from a natural source. Suitable sources of phospholipids include egg, heart (e.g. bovine), brain, liver (e.g. bovine) and plant sources including soybean. Such sources may provide one or more constituents of component ii) which may comprise any mixture of phospholipids. Any single PC or mixture of PCs from these or other sources may be used, but mixtures comprising soy PC or egg PC are highly suitable. The PC component preferably contains at least 50% soy PC or egg PC, more preferably at least 75% soy PC or egg PC and most preferably essentially pure soy PC or egg PC.

In one embodiment applicable to all aspects of the invention, component ii) comprises PC. Preferably the PC is derived from soy. Preferably the PC comprises 18:2 fatty acids as the primary fatty acid component with 16:0 and/or 18:1 as the secondary fatty acid components. These are preferably present in the PC at a ratio of between 1.5:1 and 6:1. PC having approximately 60-65% 18:2, 10 to 20% 16:0, 5-15% 18:1, with the balance predominantly other 16 carbon and 18 carbon fatty acids is preferred and is typical of soy PC.

In an alternative but equally preferred embodiment, the PC component may comprise synthetic dioleoyl PC (DOPC). This is believed to provide increased stability and so will be particularly preferable for compositions needing to be stable to long term storage, and/or having a long release period *in vivo.* In this embodiment the PC component preferably contains at least 50% synthetic dioleoyl PC, more preferably at least 75% synthetic dioleoyl PC and most preferably essentially pure synthetic dioleoyl PC. Any remaining PC is preferably soy or egg PC as above.

Synthetic or highly purified PCs, such as dioleoyl phosphatidyl choline (DOPC) are highly appropriate as all or part of component ii). The synthetic PC is most preferably 1,2-dioleoyl-sn-glycero-3-phosphocholine (DOPC), and other synthetic PC components include DDPC (1,2-Didecanoyl-sn-glycero-3-phosphocholine); DEPC(1,2-Dierucoyl-sn-glycero-3-phosphocholine); DLOPC(1,2-Dilinoleoyl-sn-glycero-3-phosphocholine); DLPC(1,2-Dilauroyl-sn-glycero-3-phosphocholine); DMPC(1,2-Dimyristoyl-sn-glycero-3-phosphocholine);); DPPC(1,2-Dipalmitoyl-sn-glycero-3 -phosphocholine); DSPC(1,2-Distearoyl-sn-glycero-3-phosphocholine); MPPC(1-Myristoyl-2-palmitoyl-sn-glycero 3-phosphocholine); MSPC(1-Myristoyl-2-stearoyl-sn-glycero-3-phosphocholine); PMPC(1-Palmitoyl-2-myristoyl-sn-glycero-3-phosphocholine); POPC(1-Palmitoyl-2-oleoyl-sn-glycero-3-phosphocholine); PSPC(1-Palmitoyl-2-stearoyl-sn-glycero-3-phosphocholine); SMPC(1-Stearoyl-2-myristoyl-sn-glycero-3-phosphocholine); SOPC(1-Stearoyl-2-oleoyl-sn-glycero-3-phosphocholine); and SPPC(1-Stearoyl-2-palmitoyl-sn-glycero-3-phosphocholine), or any combination thereof.

In some circumstances, such as in the absence of stabilising agents such as EDTA, the use of synthetic or highly purified PCs (e.g. DOPC) may provide greater stability for the active agent in the formulations. Thus in one embodiment, component ii) may comprise (e.g. may comprise at least 75%) synthetic or highly purified (e.g. purity >90%) PCs (e.g. DOPC). This may particularly be in the absence of chelating agents such as EDTA. In an alternative embodiment, component ii) may comprise (e.g. comprise at least 75%) naturally derived PCs, such as soy PC or egg PC. This will particularly be where at least one stabilising component (such as an antioxidant, chelator etc) is included in the precursor formulation.

A particularly favoured combination of components i) and ii) are mixtures of mono-, di- and tri- fatty acid esters of sorbitan with PC, especially such mixtures with soy PC and/or DOPC. Appropriate amounts of each component suitable for the combination are those amounts indicated herein for the individual components in any combination. This applies also to any combinations of components indicated herein, where context allows.

In one embodiment, phospholipid component ii) comprises dioleoyl phosphatidyl ethanolamine (DOPE), Soy PE and/or Egg PE, or mixtures of at least one of DOPE/Soy PE/Egg PE. In another embodiment component ii) comprises at least one of dioleoyl phosphatidyl ethanolamine (DOPE), Soy PE and/or Egg PE optionally as a mixture with at least one of dioleoyl phosphatidyl choline (DOPC), Soy PC (SPC), and/or Egg PC (EPC).

The phospholipid portion may be derived from a natural source. Suitable sources of phospholipids include egg, heart (e.g. bovine), brain, liver (e.g. bovine), milk and plant sources including soybean. Particularly preferred are Soy and Egg phospholipids, especially Soy PE and/or Egg PE. Such sources may provide one or more constituents of component ii) which may comprise any mixture of phospholipids. Preferably component ii) comprises Soy PE and/or Egg PE.

In one embodiment, the phospholipid component ii) (as a whole) forms a reversed hexagonal liquid crystalline phase at 37°C in the presence of excess aqueous phase, for example excess water.

By carefully controlling the ratio of components i) : ii) it is possible to dispense with the need for a liquid crystal hardener in the depot precursor formulations (pre-formulations) of the invention. The ratio by wt.% of components i) : ii) is in the range of 30:70 to 80:20, preferably 35:65 to 75:35, more preferably 45:55 to 75:25, such as around 60:40.

In another embodiment component i) comprises or consists of mixtures of mono-, di- and tri- fatty acid esters of sorbitan and component ii) comprises or consists of soy PC. In this embodiment the preferred ratio of i) : ii) is 45:55 to 75:25, preferably 50:50 to 75:25, more preferably 55:45 to 70:30, such as 60:40 to 65:35.

In an alternative embodiment component i) comprises or consists of mixtures of mono-, di- and tri- fatty acid esters of sorbitan and component ii) comprises or consists of DOPE. In this embodiment the preferred ratio of i) : ii) is 30:70 to 75:25, preferably 40:60 to 70:30, such as 50:50 to 60:40.

As used herein, terms "around", "about", "approximately", etc. take their natural meaning, in that the specified value is the most preferred disclosure but values close to this are also suitable. In particular, values of ± 10% of the specified value may be encompassed by such terms, preferably ± 5% and most preferably ± 2%. Where a composition is said to "comprise" a particular component then this indicates that other components may also be present. Where a composition is said to "consist essentially of' a particular component or set of components then this indicates that the specified components control the essence of the composition and thus will be the dominant components. This may indicate that the composition is made up to at least 90 wt% of the specified components, preferably at least 95% and most preferably at least 98 wt%.

The lower wt.% limit of component ii) in the pre-formulation is generally around 20 wt.%, preferably more than 30 wt.%, preferably more than 35 wt.%, more preferably more than 40 wt.%. The upper wt.% limit of component ii) in the pre-formulations is around 80 wt.%, preferably less than 70 wt.%, more preferably below 60 wt.%.

Typically, the amount of component ii) in the pre-formulation as a whole, or the sum of components ii) in the case of a mixture of phospholipids, will be 20-70 wt.%, preferably 25-60 wt.%, more preferably 30-60 wt.%.

### Component iii) - Solvent

Component iii) of the pre-formulations of the invention comprises, consists essentially of, or consists of an oxygen containing, low viscosity organic solvent. Since the pre-formulation is to generate a depot composition following administration (e.g. *in vivo*)*,* upon contact with an aqueous fluid, it is desirable that this solvent be tolerable to the subject and be capable of mixing with the aqueous fluid, and/or diffusing or dissolving out of the pre-formulation into the aqueous fluid. Solvents having at least moderate water solubility are thus preferred.

In a preferred embodiment, the solvent is such that a relatively small addition to the composition comprising a and b, i.e. below 20% (by wt%), or more preferably below 10%, give a large viscosity reductions of one order of magnitude or more. As described herein, the addition of 10% solvent can give a reduction of two, three or even four orders of magnitude in viscosity over the solvent-free composition, even if that composition is a solution or L₂ phase containing no solvent, or an unsuitable solvent such as water (subject to the special case considered below), or glycerol. Typical solvents suitable for use as component iii) include at least one solvent selected from alcohols, ketones, esters (including lactones), ethers, amides (including lactams) and sulphoxides. Examples of suitable alcohols include ethanol and isopropanol. Monools are preferred to diols and polyols. Where diols or polyols are used, this is preferably in combination with an at least equal amount of monool or other preferred solvent. Examples of ketones include acetone and propylene carbonate. Suitable ethers include diethylether, glycofurol, diethylene glycol monoethyl ether, dimethylisobarbide, and polyethylene glycols. Suitable esters include ethyl acetate and isopropyl acetate and dimethyl sulphide is as suitable sulphide solvent. Suitable amides and sulphoxides include N-methyl pyrrolidone (NMP), 2-pyrrolidone, dimethylformamaide (DMF), dimethylacetamide (DMA) and dimethylsulphoxide (DMSO), respectively. Less preferred solvents include dimethyl isosorbide, tetrahydrofurfuryl alcohol, diglyme and ethyl lactate.

Since the pre-formulations are to be administered to a living subject, it is necessary that the solvent component iii) is sufficiently biocompatible. The degree of this biocompatibility will depend upon the application method and since component iii) may be any mixture of solvents, a certain amount of a solvent that would not be acceptable in large quantities may evidently be present. Overall, however, the solvent or mixture forming component iii) must not provoke unacceptable reactions from the subject upon administration. Generally such solvents will be hydrocarbons or preferably oxygen containing hydrocarbons, both optionally with other substituents such as nitrogen containing groups. It is preferable that little or none of component iii) contains halogen substituted hydrocarbons since these tend to have lower biocompatibility. Where a portion of halogenated solvent such as dichloromethane or chloroform is necessary, this proportion will generally be minimised. Where the depot composition is to be formed non-parenterally a greater range of solvents may evidently be used than where the depot is to be parenteral.

Component iii) as used herein may be a single solvent or a mixture of suitable solvents but will be of low viscosity. This is important because one of the key aspects of the present invention is that it provides pre-formulations that are of low viscosity and a primary role of a suitable solvent is to reduce this viscosity. This reduction will be a combination of the effect of the lower viscosity of the solvent and the effect of the molecular interactions between solvent and lipid composition. One observation of the present inventors is that the oxygen-containing solvents of low viscosity described herein have highly advantageous and unexpected molecular interactions with the lipid parts of the composition, thereby providing a non-linear reduction in viscosity with the addition of a small volume of solvent.

The viscosity of the "low viscosity" solvent component iii) (single solvent or mixture) should typically be no more than 18 mPas at 20°C. This is preferably no more than 15 mPas, more preferably no more than 10 mPas and most preferably no more than 7 mPas at 20°C.

The pre-formulation when taken as a whole has a viscosity of below 5000 mPas at 20 °C, preferably below 2000 mPas, preferably below 1000 mPas, more preferably below 600 mPas. A typical range of suitable viscosities would be, for example, 0.1 to 5000 mPas, preferably 1 to 1000 mPas, more preferably 10 to 750 mPas and most preferably 25 to 500 mPas at 20°C.

The solvent component iii) will generally be at least partially lost upon *in vivo* formation of the depot composition, or diluted by absorption of water from the surrounding air and/or tissue. It is preferable, therefore, that component iii) be at least to some extent water miscible and/or dispersible and at least should not repel water to the extent that water absorption is prevented. In this respect also, oxygen containing solvents with relatively small numbers of carbon atoms (for example up to 10 carbons, preferably up to 8 carbons) are preferred. Obviously, where more oxygens are present a solvent will tend to remain soluble in water with a larger number of carbon atoms. The carbon to heteroatom (e.g. N, O, preferably oxygen) ratio will thus often be around 1:1 to 6:1, preferably 2:1 to 4:1. Where a solvent with a ratio outside one of these preferred ranges is used then this will preferably be no more than 75%, preferably no more than 50%, in combination with a preferred solvent (such as ethanol). This may be used, for example to decrease the rate of evaporation of the solvent from the pre-formulation in order to control the rate of liquid crystalline depot formation.

Preferably, component iii) is selected from alcohols, ketones, esters , ethers, amides, sulphoxides and mixtures thereof. More preferably component iii) is selected from monool alcohols, diols, triols, ethers, ketones and amides. Most preferred solvents for component iii) are selected from the group consisting of low molecular weight PEGs (200-500 Dalton), ethanol, NMP, DMSO or mixtures thereof. Especially preferred are ethanol, DMSO and NMP as well as mixtures thereof.

Pre-formulations of the invention form liquid crystalline phases spontaneously upon contact with excess water, and it will be appreciated therefore that the loading of components in organic solvent is not especially critical. However, it is obviously desirable to reduce the level of organic solvent to reduce the dosage volume, particularly for applications which require parenteral administration, such as injection. It is preferred that the wt.% of solvent is below 50 wt.%, preferably below 40 wt.%, more preferably below 25 wt.%, preferably below 20 wt.%. Preferred levels are 15 wt.% or below.

### Bioactive agents / Active pharmaceutical ingredients

The nature of the components of the pre-formulations of the present invention is that the components are typically highly biocompatible. The precursor formulations are typically used to form a "depot" for the controlled release of at least one bioactive agent. Thus, in one embodiment, the optional bioactive agent may be absent from any of the formulations described herein, where context allows.

The pre-formulations of the present invention preferably contain one or more bioactive agents (described equivalently as "active agents" herein). Active agents may be any compound having a desired biological or physiological effect, such as a peptide, protein, drug, antigen, nutrient, cosmetic, fragrance, flavouring, diagnostic, pharmaceutical, vitamin, or dietary agent and will be formulated at a level sufficient to provide an *in vivo* concentration at a functional level (including local concentrations for topical compositions). Under some circumstances one or more of components i), ii) and/or iii) may also be an active agent, although it is preferred that the active agent should not be one of these components. Most preferred active agents are pharmaceutical agents including drugs, vaccines, and diagnostic agents.

Drug agents that may be delivered by the present invention include drugs which act on cells and receptors, peripheral nerves, adrenergic receptors, cholinergic receptors, the skeletal muscles, the cardiovascular system, smooth muscles, the blood circulation system, endocrine and hormone system, blood circulatory system, synoptic sites, neuroeffector junctional sites, the immunological system, the reproductive system, the skeletal system, autacoid system, the alimentary and excretory systems, the histamine system, and the central nervous system.

Examples of drugs which may be delivered by the composition of the present invention include, but are not limited to, antibacterial agents, immune modulating agents, including immunostimulants and immunosuppressants, anticancer and/or antiviral drugs such as nucleoside analogues, paclitaxel and derivatives thereof, anti inflammatory drugs/agents, such as non-steroidal anti inflammatory drugs and corticosteroids, cardiovascular drugs including cholesterol lowering and blood-pressure lowing agents, analgesics, anti-emetics including histamine HI, NK1 and 5-HT₃ receptor antagonists, corticosteroids and cannabinoids, antipsychotics and antidepressants including serotonin uptake inhibitors, prostaglandins and derivatives, vaccines, and bone modulators. Diagnostic agents include radionuclide labelled compounds and contrast agents including X-ray, ultrasound and MRI contrast enhancing agents. Nutrients include vitamins, coenzymes, dietary supplements etc.

Particularly suitable active agents include those which would normally have a short residence time in the body due to rapid breakdown or excretion and those with poor oral bioavailability. These include peptide, protein and nucleic acid based active agents, hormones and other naturally occurring agents in their native or modified forms. By administering such agents in the form of a depot composition formed from the pre-formulation of the present invention, the agents are provided at a sustained level for a length of time which may stretch to days, weeks or even several months in spite of having rapid clearance rates. This offers obvious advantages in terms of stability and patient compliance over dosing multiple times each day for the same period. In one preferred embodiment, the active agent thus has a biological half life (upon entry into the blood stream) of less than 1 day, preferably less than 12 hours and more preferably less than 6 hours. In some cases this may be as low as 1-3 hours or less. Suitable agents are also those with poor oral bioavailability relative to that achieved by injection, for where the active agent also or alternatively has a bioavailability of below 20%, or preferably below 2%, especially below 0.2%, and most preferably below 0.1% in oral formulations.

Peptide and protein based active agents include human and veterinary drugs selected from the group consisting of adrenocorticotropic hormone (ACTH) and its fragments, angiotensin and its related peptides, antibodies and their fragments, antigens and their fragments, atrial natriuretic peptides, bioadhesive peptides, bradykinins and their related peptides, calcitonin peptides including calcitonin and amylin and their related peptides, vasoactive intestinal peptides (VIP) including growth hormone releasing hormone (GHRH), glucagon, and secretin, opioid peptides including proopiomelanocortin (POMC) peptides, enkephalin pentapeptides, prodynorphin peptides and related peptides, pancreatic polypeptide-related peptides like neuropeptide (NPY), peptide YY (PYY), pancreatic polypeptide (PPY), cell surface receptor protein fragments, chemotactic peptides, cyclosporins, cytokines, dynorphins and their related peptides, endorphins and P-lidotropin fragments, enkephalin and their related proteins, enzyme inhibitors, immunostimulating peptides and polyaminoacids, fibronectin fragments and their related peptides, gastrointestinal peptides, gonadotrophin-releasing hormone (GnRH) agonists and antagonist, glucagon-like peptides 1 and 2, growth hormone releasing peptides, immunostimulating peptides, insulins and insulin-like growth factors, interleukins, luthenizing hormone releasing hormones (LHRH) and their related peptides (which are equivalent to GnRH agonists as described below), melanocortin receptor agonists and antagonists, melanocyte stimulating hormones and their related peptides, nuclear localization signal related peptides, neurotensins and their related peptides, neurotransmitter peptides, opioid peptides, oxytocins, vasopressins and their related peptides, parathyroid hormone and its fragments, protein kinases and their related peptides, somatostatins and their related peptides, substance P and its related peptides, transforming growth factors (TGF) and their related peptides, tumor necrosis factor fragments, toxins and toxoids and functional peptides such as anticancer peptides including angiostatins, antihypertension peptides, anti-blood clotting peptides, and antimicrobial peptides; selected from the group consisting of proteins such as immunoglobulins, angiogenins, bone morphogenic proteins, chemokines, colony stimulating factors (CSF), cytokines, growth factors, interferons (Type I and II), interleukins, leptins, leukaemia inhibitory factors, stem cell factors, transforming growth factors and tumor necrosis factors. An interesting class of bioactive agents suitable for the invention are peptide hormones, including those of the: glycoprotein hormone family (the gonadotropins (LH, FSH, hCG), thyroid stimulating hormone (TSH); proopiomelanocortin (POMC) family, adrenocorticotropic hormone (ACTH); the posterior pituitary hormones including vasopressin and oxytocin, the growth hormone family including growth hormone (GH), human chorionic somatomammotropin (hCS), prolactin (PRL), the pancreatic polypeptide family including PP, PYY and NPY; melanin-concentrating hormone, (MCH); the orexins; gastrointestinal hormones and peptides including GLP-1 and GIP; ghrelin and obestatin; adipose tissue hormones and cytokines including leptin, adiponectin, and resistin; natriuretic hormones; parathyroid hormone (PTH);
the calcitonin family with calcitonin and amylin; the pancreatic hormones including insulin, glucagon and somatostatin. All synthetic peptides designed to have similar receptor affinity spectrums as the above mentioned peptides are also very suitable for the invention.

A further considerable advantage of the depot compositions of the present invention is that active agents are released gradually over long periods without the need for repeated dosing. The compositions are thus highly suitable for situations where patient compliance is difficult, unreliable or where a level dosage is highly important, such as mood-altering actives, those actives with a narrow therapeutic window, and those administered to children or to people whose lifestyle is incompatible with a reliable dosing regime and for "lifestyle" actives where the inconvenience of repeated dosing might outweigh the benefit of the active. Particular classes of actives for which this aspect offers a particular advantage include contraceptives, hormones including contraceptive hormones, and particularly hormones used in children such as growth hormone, anti-addictive agents, and drugs used in treatment of poorly compliant populations, such as patients suffering from schizophrenia, Alzheimer, or Parkinson's disease, anti-depressants and anticonvulsants

Cationic peptides are particularly suitable for use where a portion of the pre-formulation comprises an anionic amphiphile such as a fatty acid or anionic lipid, including phosphatidic acid, phosphatidylglycerol, phosphatidylserine. In this embodiment, preferred peptides include octreotide, lanreotide, calcitonin, oxytocin, interferon-beta and -gamma, interleukins 4, 5, 7 and 8 and other peptides having an isoelectric point above pH 7, especially above pH 8.

In one preferred aspect of the present invention, the composition of the invention is such that a reversed micellar cubic (I₂) phase, or a mixed phase including I₂ phase is formed upon exposure to aqueous fluids and a polar active agent is included in the composition. Particularly suitable polar active agents include peptide and protein actives, oligo nucleotides, and small water soluble actives, including those listed above. Of particular interest in this aspect are the peptide octreotide and other somatostatin related peptides, interferons alpha and beta, glucagon-like peptide 1 and glucagon-like peptide 2 receptor agonists, leuprorelin and other GnRH agonists, abarelix and other GnRH antagonists, zolendronate and ibandronate and other bisphosponates.

Since all of the µ-opioid receptor agonists of choice for the treatment of moderate-to-severe chronic pain (morphine, hydromorphone, fentanyl, methadone, oxycodone, and buprenorphine) have the same mechanism of action, their physiochemical and pharmacokinetic characteristics are more critical in determining the appropriate route of administration and product formulation to be used. For example, the short elimination half-life of opioids such as morphine, hydromorphone, and oxycodone require that these agents be administered frequently to achieve around-the-clock analgesia, which makes them excellent candidates for long acting release formulations. Fentanyl and buprenorphine undergo significant first-pass metabolism and lacks sufficient bioavailability after oral administration. Together with their high potency, fentanyl and buprenorphine are excellent candidates for the long acting injection depot formulation of the invention. Sufentanil, remifentanil, oxymorphone, dimorphone, dihydroetorphine, diacetylmorphine are other potent opioid receptor agonists suitable for the invention.

Buprenorphine is also used for maintenance treatment of opioid addiction as well as potentially also cocaine and amphetamine and met-amphetamine addiction, where current sublingual buprenorphine formulations suffer from low bioavailability, high variability and limited effect duration, resulting in issues with unpredictable dose response and withdrawal symptoms, particularly in mornings. These issues effectively addressed by using the injection depot formulation of the invention, as are problems with misuse and misdirection where the need for high sublingual doses are exploited by injection, where the effect is significantly higher for the same dose, thus facilitating misuse of the drug. Similarly, opioid antagonists can be used for treating addiction using a convenient injection depot system as provided by the invention. Suitable opiate antagonists for use with the invention are naloxone, nalmefene, and naltrexone.

Antipsychotics, including risperidone, iloperidone, paliperidone, olanzapine, ziprazidone and aripiprazole are also highly suitable for the invention in view of the potential for improved treatment compliance by patients, as well as by providing stable plasma levels over time. Similarly, the invention is useful in the treatment of dementia, Alzheimer's disease and Parkinson's disease, which adversely affect cognition. Suitable active ingredients include donepezil, rivastigmine, galantamine, and emantine, and pramipexol.

A particular advantage of the present invention when used in combination with protein/peptide active agents is that aggregation of the active agent is suppressed. In one preferred embodiment, the present invention thus provides a depot precursor and particularly a depot composition as described herein comprising at least one peptide or protein active agent wherein no more than 5% of the active agent is in aggregated form. Preferably no more than 3% is aggregated and most preferably no more than 2% (especially less than 2%) is in aggregated form. This stabilisation of non-aggregated protein is highly advantageous from the point of view of high effectiveness, low side effects and predictable absorption profile. Furthermore, it is increasingly expected that protein/peptide therapeutics will have low levels of protein aggregation in order to secure regulatory approval.

Gonadotropin-releasing hormone agonists (GnRH agonists) are synthetic peptides modelled after the hypothalamic neurohormone GnRH that interacts with the gonadotropin-releasing hormone receptor to elicit its biologic response, the release of the pituitary hormones follicle stimulating hormone (FSH) and luthenizing hormone (LH). GnRH agonists are useful in treatment of cancers that are hormonally sensitive and where a hypogonadal state decreases the chances of a recurrence. Thus they are commonly employed in the medical management of prostate cancer and have been used in patients with breast cancer. Other indication areas include treatment of delaying puberty in individuals with precocious puberty, management of female disorders that are dependent on estrogen productions. In addition, women with menorrhagia, endometriosis, adenomyosis, or uterine fibroids may receive GnRH agonists to suppress ovarian activity and induce a hypoestrogenic state.

Gonadotropin-releasing hormone receptor agonists (GnRH-RAs), such as leuprolide (or leuprorelin), goserelin, histrelin, triptorelin, buserelin, deslorelin, nafarelin and related peptides are used or indicated for the treatment of a variety of conditions where they are typically administered over an extended period. GnRH-RAs form a preferred group of active agents for use in the present invention.

GnRH itself is a post-translationally modified decapeptide of structure pyro-Glu-His-Trp-Ser-Tyr-Gly-Leu-Arg-Pro-Gly-NH₂ (GnRH-I). Two natural varients are also known, GNRH-II having 5-His, 7-Trp, 8-Tyr substitutions and GnRH_III having 7-Trp, 8-Leu. Several peptide analogues with agonistic properties are known, most of which have the10-Gly-NH₂ replaced with N-Et-NH₂. Fertirelin has 10-Gly to N-Et-NH₂ substitution only, while analogues having additional substitutions over GnRH-I include Leuprorelin (Leuprolide), (6-D-Leu), Buserelin (6-Ser(Bu')), Histrelin (6-d-His(Imbzl)), Deslorelin (6-d-Trp). Another common nona-peptide agonist is Goserelin which is substituted with 6-Ser(Bu') and has 10-Gly-NH₂ replaced by AzaGly-NH₂. Narafelin (6-d-Nal) and Triptorelin (6-d-Trp) both retain the 10-Gly-NH₂ group. The structures of the two most common GnRH agonists (Leuprolide and Goserelin) are shown below as acetate salts.

Leuprolide: pyro-Glu-His-Trp-Ser-Tyr-D-Leu-Leu-Arg-Pro- N-Et-NH₂ (acetate)

Goserelin: pyro-Glu-His-Trp-Ser-Tyr-D-Ser(Bu^{t})-Leu-Arg-Pro-Azgly-NH₂ (acetate)

A small number of GnRH antagonists are also known, again based on the GnRH-I structure. These include Abarelix (D-Ala-D-Phe-D-Ala-Ser-Tyr-D-Asp-Leu-Lys(ⁱPr)-Pro-D-Ala), Antarelix (D-Nal-D-Phe-D-Pal-Ser-Phe-D-Hcit-Leu-Lys(ⁱPr)-Pro-D-Ala); Cetrorelix (D-Nal-D-Phe-D-Pal-Ser-Tyr-D-Cit-Leu-Arg-Pro-D-Ala), Ganirelix (D-Nal-D-Phe-D-Pal-Ser-Tyr-D-hArg-Leu-HArg-Pro-D-Ala), Itrelix (D-Nal-D-Phe-D-Pal-Ser-NicLys-D- NicLys -Leu-Lys(ⁱPr)-Pro-D-Ala) and Nal-Glu (D-Nal-D-Phe-D-Pal-Ser-D-Glu-D- Glu -Leu-Arg-Pro-D-Ala).

Administration of single doses of a GnRH agonist, such as leuprolide, stimulates pituitary release of gonadotropins (i.e., LH and FSH), resulting in increased serum LH and FSH concentrations and stimulation of ovarian and testicular steroidogenesis. Transient increases in serum testosterone and dihydrotestosterone (DHT) in males and in serum estrone and estradiol concentrations in premenopausal females are observed during initial therapy with single daily doses of the drug.

Although the effect of a potent GnRH agonist during short-term and/or intermittent therapy is stimulation of steroidogenesis, the principal effect of the drug in animals and humans during long-term administration is inhibition of gonadotropin secretion and suppression of ovarian and testicular steroidogenesis. The exact mechanism(s) of action has not been fully elucidated, but continuous therapy with a GnRH agonist apparently produces a decrease in the number of pituitary GnRH and/or testicular LH receptors, resulting in pituitary and/or testicular desensitization, respectively. The drug does not appear to affect receptor affinity for gonadotropins. Leuprolide's mechanism of action may also involve inhibition and/or induction of enzymes that control steroidogenesis. Other mechanisms of action may include secretion of an LH molecule with altered biologic activity or impairment of normal pulsatile patterns of LH and FSH secretion.

A number of serious medical indications are related to and/or affected by the concentration of gonadal steroid hormones. These include certain neoplastic diseases, including cancers, especially of the breast and prostate, and benign prostatic hypertrophy; premature or delayed puberty in adolescents; hirsuitism; alzheimer's disease; and certain conditions relating to the reproductive system, such as hypogonadism, anovulation, amenorrhea, oligospermia, endometriosis, leiomyomata (uterine fibroids), premenstrual syndrome, and polycystic ovarian disease. Control of this system is also important in *in vitro* fertilisation methods.

Although treatment with a GnRH agonist might be expected to exacerbate conditions affected by gonadal steroid hormone concentration, the down-regulation effect discussed above results in the decrease of these hormones to castrate level if therapy is continued for around 2 weeks or longer. As a result, hormone-receptive tumours such as certain prostate and breast cancer, as well as precocious puberty and many of the other conditions mentioned above can be improved or palliated by long-term GnRH agonist therapy.

The pre-formulations of the present invention contain one or more GnRH analogues or other active (see above) (which are intended by any reference to "active agents" herein). Since GnRH is a peptide hormone, typical GnRH analogues will be peptides, especially of 12 or fewer amino acids. Preferably such peptides will be structurally related to GnRH I, II and/or III, and/or one or more of the known analogues, including those listed here. Peptides may contain only amino acids selected from those 20 α-amino acids indicated in the genetic code, or more preferably may contain their isomers and other natural and non-natural amino acids, (generally α, β or γ amino acids) and their analogues and derivatives. Preferred amino acids include those listed above as constituents of the known GnRH analogues.

Amino acid derivatives are especially useful at the termini of the peptides, where the terminal amino or carboxylate group may be substituted by or with any other functional group such as hydroxy, alkoxy, carboxy, ester, amide, thio, amido, amino, alkyl amino, di- or tri-alkyl amino, alkyl (by which is meant, herein throughout C₁-C₁₂ alkyl, preferably C₁-C₆ alkyl e.g. methyl, ethyl, n-propyl, isopropyl, n-butyl, iso-, sec- or t-butyl etc.), aryl (e.g phenyl, benzyl, napthyl etc) or other functional groups, preferably with at least one heteroatom and preferably having no more than 10 atoms in total, more preferably no more than 6.

Particularly preferred GnRH analogues are constrained peptides of 6 to 12 alpha-amino acids, of which particular examples include those indicated above, and particularly leuprolide and goserelin, of the sequences indicated above.

By "GnRH analogues", as used herein is indicated any GnRH agonist or antagonist, preferably peptides, peptide derivatives or peptide analogues. Peptide derived GnRH agonists are most preferred, such as those indicated above and especially leuprolide or goserelin.

The GnRH analogue will generally be formulated as 0.02 to 12% by weight of the total formulation. Typical values will be 0.1 to 10%, preferably 0.2 to 8% and more preferably 0.5 to 6%. A GnRH analogue content of around 1-5% is most preferable.

Doses of the GnRH analogue suitable for inclusion in the formulation, and thus the volume of formulation used will depend upon the release rate (as controlled, for example by the solvent type and amount use) and release duration, as well as the desired therapeutic level, the activity of the specific agent, and the rate of clearance of the particular active chosen. Typically an amount of 0.1 to 500 mg per dose would be suitable for providing a therapeutic level for between 7 and 180 days. This will preferably be 1 to 200 mg. For leuprolide or goserelin, the level will typically be around 1 to 120 mg (e.g. for a 30 to 180 day duration). Preferably, the amount of leuprolide will be around 0.02 to 1 mg per day between injections, for depots designed for release over 30 days to 1 year, preferably 3 to 6 months. Evidently, the stability of the active and linearity of the release rate will mean that the loading to duration may not be a linear relationship. A depot administered every 30 days might have, for example 2 to 30 mg or a 90 day depot have 6 to 90 mg of active, such as one of the GnRH analogues indicated herein.

Where the active agent comprises a 5HT₃ antagonist or second generation 5HT₃ antagonist, this is preferably selected from odansetron, tropisetron, granisetron, dolasetron, palonosetron, alosetron, cilansetron and/or ramosetron or mixtures thereof. Doses of the 5HT₃ antagonist suitable for inclusion in the formulation, and thus the volume of formulation used will depend upon the release rate (as controlled, for example by the solvent type and amount use) and release duration, as well as the desired therapeutic level, the activity of the specific agent, and the rate of clearance of the particular active chosen. Typically an amount of 1 to 500 mg per dose would be suitable for providing a therapeutic level for between 5 and 90 days. This will preferably be 1 to 300 mg. For granisetron, the level will typically be around 10 to 180 mg (e.g. for a 3 to 60 day duration). Preferably, the amount of granisetron will be around 0.2 to 3 mg per day between injections, for depots designed for release over 30 days to 1 year, preferably 3 to 6 months. Evidently, the stability of the active and linearity of the release rate will mean that the loading to duration may not be a linear relationship. A depot administered every 30 days might have, for example 2 to 30 mg or a 90 day depot have 6 to 90 mg of active.

Somatostatins (Growth Hormone Release Inhibiting Factors, SSTs) are natural peptide hormones with a wide distribution in animals, acting as neurotransmitters in the central nervous system, and having diverse paracrine/autocrine regulatory effects on several tissues. Two biologically active products are known in higher species, SST-14 and SST-28, a congener of SST-14 extended at the N-terminus.

SST-14 is a 14 residue cyclic peptide hormone having the sequence Ala-Gly-Cys-Lys-Asn-Phe-Phe-Trp-Lys-Thr-Phe-Thr-Ser-Cys, where the two cysteine residues are connected by a disulphide bridge to generate a type II β-turn at the key binding sequence of Phe-Trp-Lys-Thr. The biological half-life of natural SST-14 is very short (1-3 minutes) and so it is not, in itself, a viable therapeutic in current formulations, but an increasing number of somatostatin receptor agonists are becoming available with higher activities and/or longer clearance times *in vivo.*

Somatostatin receptor agonists (SRAs), such as SST-14, SST-28, octreotide, lanreotide, vapreotide, pasireotide (SOM230) and related peptides, are used or indicated in the treatment of a variety of conditions where they are typically administered over an extended period. SRAs form a preferred group of active agents for use in the present invention.

Octreotide, for example, is the synthetic octapeptide with sequence D-Phe-Cys-Phe-D-Trp-Lys-Thr-Cys-Thr-ol (2-7 disulphide bridge) and is typically administered as an acetate salt. This SST-14 derivative retains the key Phe-(D)Trp-Lys-Thr β-turn required for *in vivo* SST-like activity but, in contrast to the natural hormone, has a terminal half-life of around 1.7 hours. Octreotide is used in treatment of conditions including carcinoid tumours and acromegaly, and is typically administered over a sustained period of weeks, or more commonly many months or years. Somatostatin receptor agonists are of particular interest for the treatment of many different types of cancers since a wide variety of tumours are found to express somatostatin receptors (SSTRs). There are five known types of SSTRs (SSTR1-SSTR5), showing equally high affinity for SST-14. The most investigated somatostatin receptor agonists, including octreotide, show high selectivity for SSTR2 and SSTR5; thus, octreotide is of particular interest for the treatment of tumours expressing these types of receptors.

The most common "simple" formulation of Octreotide is "Sandostatin" (RTM) from Novartis. This is an aqueous solution for subcutaneous (s.c) injection, and a 100 µg dose reaches a peak concentration of 5.2 ng/ml at 0.4 hours post injection. The duration of action can be up to 12 hours but s.c. dosing is generally carried out every 8 hours. Evidently, s.c. injection 3 times daily for periods of months or years is not an ideal dosing regime.

Pasireotide is a multireceptor-targeted somatostatin analogue with high affinity for somatostatin receptor subtypes sstr1,2,3 and sstr5 that has been developed for the treatment of neuroendocrine diseases. Two formulations of pasireotide have currently been developed: an immediate-release formulation for subcutaneous (sc) injection and a long-acting-release (LAR) formulation.

Pasireotide was initially developed by Novartis Pharma as a treatment for Cushing's disease/syndrome and acromegaly, but has potential applicability in the treatment of several conditions for which somatostatin analogues such as octreotide are indicated, including carcinoid tumours.

Following a single subcutaneous dose of pasireotide, human plasma levels typically peak quickly, at around 15 minutes to 1 hour after dosing, with an initial half-life of 2-3 hours following that peak. Although clearance half-life is greater for later phases of the decline, it is clear that the Cmax/Cave for such a delivery will be rather high.

Pasireotide LAR is a long acting formulation of pasireotide which addresses some of the above issues. However, this is a polymer microparticle based system with the inherent limitations of such a system, as are known in the art and described herein above.

Carcinoid tumours are intestinal tumour arising from specialised cells with paracrine functions (APUD cells). The primary tumour is commonly in the appendix, where it is clinically benign. Secondary, metastatic, intestinal carcinoid tumours secrete excessive amounts of vasoactive substances, including serotonin, bradykinin, histamine, prostaglandins, and polypeptide hormones. The clinical result is carcinoid syndrome (a syndrome of episodic cutaneous flushing, cyanosis, abdominal cramps, and diarrhea in a patient with valvular heart disease and, less commonly, asthma and arthropathy). These tumours may grow anywhere in the gastrointestinal tract (and in the lungs) with approximately 90% in the appendix. The remainder occurs in the ileum, stomach, colon or rectum. Currently, treatment of carcinoid syndrome starts with i.v. bolus injection followed by i.v. infusion. When sufficient effect on symptoms has been established, treatment with a depot formulation of octreotide formulated in poly lactic-co-glycolic acid (PLGA) microspheres is started. However, during the first two weeks or more after injection of the depot, daily s.c. injections with octreotide are recommended to compensate for the slow release from the PLGA spheres.

Certain of the pre-formulations of the present invention contain salts of one or more somatostatin receptor agonists (which are preferred examples of the peptide actives, which in turn are intended by any reference to "active agents" herein). Since SST-14 is a peptide hormone, typical somatostatin receptor agonists will be peptides, especially of 14 or fewer amino acids. Preferably such peptides will be structurally constrained such as by being cyclic and/or having at least one intra-molecular crosslink. Amide, ester or particularly disulphide crosslinks are highly suitable. Preferred constrained peptides will exhibit a type-2 β turn. Such a turn is present in the key region of somatostatin. Peptides may contain only amino acids selected from those 20 α-amino acids indicated in the genetic code, or more preferably may contain their isomers and other natural and non-natural amino acids, (generally α, β or γ, L- or D-amino acids) and their analogues and derivatives. The term "somatostatin receptor agonist" as used herein may optionally also encompass SST-14 and/or SST-28, since these are viable peptide actives when formulated as salts in the very high performance slow-release formulations described herein.

Amino acid derivatives and amino acids not normally used for protein synthesis are especially useful at the termini of the peptides, where the terminal amino or carboxylate group may be substituted by or with any other functional group such as hydroxy, alkoxy, ester, amide, thio, amino, alkyl amino, di- or tri-alkyl amino, alkyl (by which is meant, herein throughout C₁-C₁₈ alkyl, preferably C₁-C₈ alkyl e.g. methyl, ethyl, n-propyl, isopropyl, n-butyl, iso-, sec- or t-butyl etc.), aryl (e.g phenyl, benzyl, napthyl etc) or other functional groups, preferably with at least one heteroatom and preferably having no more than 10 atoms in total, more preferably no more than 6.

Particularly preferred somatostatin receptor agonists are constrained peptides of 6 to 10 α-amino acids, of which particular examples include octreotide, lanreotide and its cyclic derivative of sequence, both having a Cys-Cys intramolecular disulphide crosslink, pasireotide and vapreotide. Most preferred are octreotide and pasireotide.

The somatostatin receptor agonist, if present, will generally be formulated as 0.1 to 10% by weight of the total formulation. Typical values will be 0.5 to 9%, preferably 1 to 8% and more preferably 1 to 7%. A somatostatin receptor agonist content of 2-5 % is most preferable.

Doses of the somatostatin receptor agonist suitable for inclusion in the formulation, and thus the volume of formulation used, will depend upon the release rate (as controlled, for example by the solvent type and amount use) and release duration, as well as the desired therapeutic level, the activity and the rate of clearance of the particular active chosen. Typically an amount of 1 to 500 mg per dose would be suitable for providing a therapeutic level for between 7 and 90 days. This will preferably be 5 to 300 mg. For octreotide, the level will typically be around 10 to 180 mg (e.g. for a 30 to 90 day duration). Preferably, the amount of octreotide will be around 0.2 to 3 mg per day between injections. Thus a depot administered every 30 days would have 6 to 90 mg or a 90 day depot have 18 to 270 mg of octreotide.

For Pasireotide, the dosage would typically be an amount of around 0.05 to 40 mg per week of depot duration, preferably 0.1 to 20 mg per week duration (e.g. 1 to 5 mg per week) for a duration of 1 to 24 weeks, preferably 2 to 16 (e.g. 3, 4, 8, 10 or 12) weeks. In an alternative embodiment the pre-formulation may be formulated for dosing weekly (e.g. every 7±1 days). A total dose of 0.05 to 250 mg of Pasireotide per dose would be suitable for providing a therapeutic level for between 7 and 168 days. This will preferably be 0.1 to 200 mg, e.g. 0.2 to 150 mg, 0.1 to 100 mg, 20 to 160 mg etc. Evidently, the stability of the active and effects on the release rate will mean that the loading to duration may not be a linear relationship. A depot administered every 30 days might have, for example 0.2 to 20 mg of Pasireotide, or a 90 day depot might have 30 to 60 mg of Pasireotide.

Where the salt of a peptide active agent, such as an SRA, is used in the formulations of the present invention, this will be a biologically tolerable salt. Suitable salts include the acetate, pamoate, or chloride salts. The chloride salt is most preferred.

The amount of bioactive agent to be formulated with the pre-formulations of the present invention will depend upon the functional dose and the period during which the depot composition formed upon administration is to provide sustained release. Typically, the dose formulated for a particular agent will be around the equivalent of the normal daily dose multiplied by the number of days the formulation is to provide release. Evidently this amount will need to be tailored to take into account any adverse effects of a large dose at the beginning of treatment and so this will generally be the maximum dose used. The precise amount suitable in any case will readily be determined by suitable experimentation.

Preferably, when present, the pre-formulation of the invention will comprise 0.1-10 wt.% of said active agent by weight of components i) + ii) + iii).

Preferably the active agent where present is selected from:
interferons; GnRH agonists buserelin, deslorelin, goserelin, leuprorelin/leuprolide,
naferelin and triptorelin; GnRH antagonists, e.g. cetrorelix, ganirelix, abarelix,
degarelix; glucagon-like peptide-1 (GLP-1) and analogues thereof, e.g. GLP-1(7-37), GLP-1(7-36) amide, liraglutide, exenatide, and lixisenatide (AVE0010);
glucagon-like peptide 2 agonists (GLP-2) and analogues thereof, e.g. GLP-2 and Elsiglutide (ZP1846); DPPIV inhibitors; somatostatins SST-14 and SST-28 and somatostatin receptor (SSTR) agonists, e.g. octreotide, lanreotide, vapreotide, pasireotide.

Other peptides suitable for the invention include: angiopeptin, angiotensin I, II, III, antileukinate, anti-inflammatory peptide 2, aprotinin, bradykinin, bombesin, calcitonin, calcitriol, cholecystokinin (CCK), colony-stimulating factor, corticotropin-releasing factor, C-Peptide, DDAVP, dermorphin-derived tetrapeptide (TAPS), dynorphin, endorphins, endostatin, endothelin, endothelin-1, enkephalins, epidermal growth factor, erythropoietin, fibroblast growth factor, follicle stimulating hormone, follistatin, follitropin, galanin, galanin-like peptide, galectin-1, gastrin, gastrin-releasing peptide, G-CSF, ghrelin, glial-derived neurotrophic factor, GM-CSF, granulocyte colony-stimulating factor, growth hormone, growth hormone-releasing factor, hepatocyte growth factor, insulin, insulin-like growth factors-I and I, interferons, interleukins, leptin, leukemia inhibitory factor, melanocortin 1, 2, 3, 4, melanocyte-stimulating hormone metastin, monocyte chemotactic protein-1 (MCP-1), morphiceptin, NEP1-40, neuropeptide Y, neuropeptide W, orexin-A & orexin-B, oxytocin p21-Cip1/WAF-1, TAT fusion protein, parathyroid hormone, pigment epithelium-derived growth factor (PEDF), peptide, peptide, prorenin handle region, peptide YY (3-36), platelet activating factor, platelet-derived growth factor, prorenin decapeptide, protegrin-1, PR39, prolactin, relaxin, secretin, substance P, tumor necrosis factor, urocortin, vascular endothelial growth factor, vasoactive intestinal polypeptide, vasopressin.

Most preferably the active agent is at least one selected from buprenorphine, octreotide, pasireotide, leuprolide and goserelin. For example, at least one selected from buprenorphine, leuprolide and goserelin.

In one embodiment, applicable to all aspects of the invention, the active agent excludes somatostatin receptor agonists, in other words the active agent does not comprise any somatostatin receptor agonist.

In a further embodiment, the active agent, when present, may exclude certain specific somatostatin receptor agonists, namely pasireotide, octreotide and/or salts and mixtures thereof. In this embodiment, the active agent may comprise somatostatin receptor agonists with the exception of pasireotide, octreotide and/or salts and mixtures thereof.

If a bioactive agent is present then it is preferred that this component does not act as a liquid crystal hardener, i.e. the bioactive agent does not contribute to the degree of curvature of the lipid membrane.

In one aspect of the invention, pre-formulations of the invention may exclude non-peptide active agents, since non-peptide active agents are more likely to interact strongly with lipid and thereby contribute to the degree of curvature of the membrane.

In a most preferred aspect pre-formulations of the invention may comprise a peptide active agent. In another embodiment both peptide and non-peptide active agents are excluded from pre-formulations of the invention.

The term 'liquid crystal hardener' as used herein encompasses all embodiments of the term described in WO2013/032207 A1. Specifically, the term 'liquid crystal hardener' refers to any component being free of an ionizable group, having a hydrophobic moiety of 15 to 40 carbon atoms and having a triacyl group or a carbon ring structure. It is preferable, however, that triacyl fatty acid esters of sorbitan are excluded from the definition of "liquid crystal hardener". Such triacyl esters will typically be present in at least a small proportion in component i) and are thus not an added Liquid Crystal Hardener. Ionizable groups include carboxyl groups or amine groups. Hydroxy groups are not considered to be ionizable groups for the purposes of the present invention.

It will be appreciated that the definition 'liquid crystal hardener' may encompass compounds which also fall within the scope of components i) or ii), i.e. a sorbitan ester, or a phospholipid. For the purposes of the present invention, it is a requirement that the liquid crystal hardener must also be distinct from the categories of sugar- or sugar derivative ester or phospholipid. Thus, any sugar- or sugar derivative ester or phospholipid is not considered to be a liquid crystal hardener.

Retinyl palmitate, benzyl benzoate, ubiquinone, tocopherols and cholesterol or derivatives thereof are considered liquid crystal hardeners and are thus excluded from pre-formulations of the invention. The term "exclude" has the meaning explained previously and applies in all cases where context allows, especially in respect of the "crystal hardeners". The levels of retinyl palmitate, benzyl benzoate, ubiquinone, tocopherols, cholesterol or derivates thereof in the pre-formulation are below 1,000 ppm by weight relative to the composition as a whole. Preferably, these components are present below 500 ppm, more preferably below 300 ppm, still more preferably below 100 ppm. Such components may, in one embodiment, be excluded completely, such as to below the limit of detection.

Triglycerides are also excluded from pre-formulations of the invention, or the levels of triglycerides in the pre-formulation are below 1,000 ppm by weight relative to the pre-formulation as a whole.

In another embodiment non-peptide bioactive agents, especially non-peptide bioactive agents having liquid crystal hardening activity may be excluded from formulations of the invention.

In an alternative aspect pre-formulation of the invention may additionally comprise a peptide bioactive agent. It will however be understood that peptide bioactive agents are not 'liquid crystal hardeners' as used herein.

In a most preferred embodiment, the composition consists essentially of or consist of at least one sorbitan ester (or a mixture thereof), at least one phospholipid, an alcoholic solvent, optionally a polar solvent and optionally a peptide bioactive agent, according to claim 1.

These may be present at the ratios and preferred ratios indicated herein. It will be appreciated that components such as antioxidants, preservatives etc may also be present.

### Administration

As mentioned above, the pre-formulation for use may be administered and applied using a route appropriate for the condition to be treated and the bioactive agent used. The term "parenteral" as used herein is given its established meaning of "through the skin" rather than all "non-oral" routes. Thus parenteral primarily indicates administration by injection, infusion and similar techniques (such as needle-less injection). The term "non-parenteral" thus covers application routes other than through the skin. A parenteral depot will thus be formed by parenteral (e.g. injectable, such as by subcutaneous or intramuscular injection) administration.

In one embodiment, the pre-formulations of the present invention will generally be administered parenterally. This administration will generally not be an intra-vascular method but will preferably be subcutaneous intracavitary or intramuscular. Typically the administration will be by injection, which term is used herein to indicate any method in which the formulation is passed through the skin, such as by needle, catheter or needle-less injector.

In parenteral (especially subcutaneous (s.c.)) depot precursors, preferred active agents are those suitable for systemic administration including antibacterials (including amicacin, monocycline and doxycycline), local and systemic analgesics (including tramadol, fentanyl, morphine, hydromorphone, buprenorphine, methadone, oxycodone, codeine, asperine, acetaminophen), immunosuppressants (such as thalidomide, lenalidomide,, sirolimus, deforolimus, everolimus, temsirolimus, Umirolimus, zotarolimus), NSAIDS (such as ibuprofene, naproxene, keteprofene, diclofenac, indomethansine, sulindac, tolmethin, salysylic acids such as salisylamide, diflunisal), Cox1 or Cox2 inhibitors (such as celecoxib, rofecoxib, valdecoxib), oncology and endocrinology agents (including octreotide, lanreotide, buserelin, luprorelin, goserelin, triptorelin, avorelin, deslorein, abarelix, degarelix, fulvestrant, interferon alpha, interferon beta, darbepoetin alpha, epoetin alpha, beta, delta, cytarabine, docetaxel, and paclitaxel), antiemetics (like granisetron, odansetron, palonsetron, aprepitant, fosaprepitant, netupitant, dexamethasone, in particular 5HT₃ antagonists or second generation 5HT₃ antagonists, preferably selected from odansetron, tropisetron, granisetron, dolasetron, palonosetron, alosetron, cilansetron and/or ramosetron or mixtures thereof), antipsychotics (like bromperidol, risperidone, olanzapine, iloperidone, paliperadone, pipotiazine and zuclopenthixol), antivirals, anticonvulsants (for instance tiagabine topiramate or gabapentin) or nicotine, hormones (such as testosterone, testosterone cypionate, and testosterone undecanoate, medroxyprogesterone, estradiol) growth hormones (like human growth hormone), and growth factors (like granulocyte macrophage colony-stimulating factor), anti diabetic agents (such as GLP_1(7-36) amide, GLP-1(7-37), liraglutide, exenatide, lixisenatide, and glucagon), acetylcholinesterase receptor inhibitors (such as neostigmine, physostigmine, and rivastigmine), and pramipexol.

### Phase Structures

The pre-formulations of the present invention provide non-lamellar liquid crystalline depot compositions upon exposure to aqueous fluids, especially *in vivo* and in contact with body surfaces. In a preferred embodiment the liquid crystalline phases of the invention are formed *in situ.*

As used herein, the term "non-lamellar" is used to indicate a normal or reversed liquid crystalline phase (such as a cubic or hexagonal phase) or the L3 phase or any combination thereof. The term liquid crystalline indicates all hexagonal, all cubic liquid crystalline phases and/or all mixtures thereof. Hexagonal as used herein indicates "normal" or "reversed" hexagonal (preferably reversed) and "cubic" indicates any cubic liquid crystalline phase, preferably reversed.

Preferably in the pre-formulation of the invention the liquid crystalline phase structure formed upon contact with an aqueous fluid is a reversed hexagonal phase structure (H₂) and/or a reversed cubic phase structure (I₂) or a mixture or intermediates thereof. With intermediates we refer to phases with mean curvatures between the mean curvature of H₂ and I₂ phases, respectively, and which position in a phase diagram is between these two phases in case both are present. Preferably the liquid crystalline phase structure is selected from H₂, I₂ or mixtures thereof.

It is important to appreciate that the precursor formulations (pre-formulations) of the present invention are of low viscosity. As a result, these pre-formulations must not be in any bulk liquid crystalline phase since all liquid crystalline phases have a viscosity significantly higher than could be administered by syringe or similar device. The pre-formulations of the present invention will thus be in a non-liquid crystalline state, such as a molecular solution, L₂ or L₃ phase, particularly solution or L₂. The L₂ phase as used herein throughout is preferably a "swollen" L₂ phase containing greater than or about 10 wt% of solvent component iii) having a viscosity reducing effect. This is in contrast to a "concentrated" or "unswollen" L₂ phase containing no solvent, or a lesser amount of solvent, or containing a solvent (or mixture) which does not provide the decrease in viscosity associated with the oxygen-containing, low viscosity solvents specified herein.

Upon administration, the pre-formulations of the present invention undergo a phase structure transition from a low viscosity mixture to a high viscosity (generally tissue adherent) depot composition. Generally this will be a transition from a molecular mixture/solution, swollen L₂ and/or L₃ phase to one or more (high viscosity) liquid crystalline phases such as normal or reversed hexagonal or cubic liquid crystalline phases or mixtures thereof. As indicated above, further phase transitions may also take place following administration. Obviously, complete phase transition is not necessary for the functioning of the invention but at least a surface layer of the administered mixture will form a liquid crystalline structure. Generally this transition will be rapid for at least the surface region of the administered formulation (that part in direct contact with air, body surfaces and/or body fluids). This will most preferably be over a few seconds or minutes (e.g. up to 30 minutes, preferably up to 10 minutes, more preferably 5 minutes of less). The remainder of the composition may change phase to a liquid crystalline phase more slowly by diffusion and/or as the surface region disperses.

In one preferred embodiment, the present invention thus provides a pre-formulation as described herein of which at least a portion forms a hexagonal liquid crystalline phase upon contact with an aqueous fluid. The thus-formed hexagonal phase may gradually disperse, releasing the active agent, or may subsequently convert to a cubic liquid crystalline phase, which in turn then gradually disperses. It is believed that the hexagonal phase will provide a more rapid release of active agent, in particular of hydrophilic active agent, than the cubic phase structure, especially the I₂ and L₂ phase. Thus, where the hexagonal phase forms prior to the cubic phase, this will result in an initial release of active agent to bring the concentration up to an effective level rapidly, followed by the gradual release of a "maintenance dose" as the cubic phase degrades. In this way, the release profile may be controlled.

Without being bound by theory, it is believed that upon exposure (e.g. to body fluids), the pre-formulations of the invention lose some or all of the organic solvent included therein (e.g. by diffusion and/or evaporation) and take in aqueous fluid from the bodily environment (e.g. moist air close to the body or the *in vivo* environment) such that at least a part of the formulation generates a non-lamellar, particularly liquid crystalline phase structure. In most cases these non-lamellar structures are highly viscous and are not easily dissolved or dispersed into the *in vivo* environment and are bioadhesive and thus not easily rinsed or washed away.

Furthermore, because the non-lamellar structure has large polar, apolar and boundary regions, it is highly effective in solubilising and stabilising many types of active agents and protecting these from degradation mechanisms. As the depot composition formed from the pre-formulation gradually degrades over a period of days, weeks or months, the active agent is gradually released and/or diffuses out from the composition. Since the environment within the depot composition is relatively protected, the pre-formulations of the invention are highly suitable for active agents with a relatively low biological half-life (see above).

Figures:
Figure 1 illustrates formulation viscosity against the proportion of phospholipid in respect to total lipid content.
Figure 2 shows synchrotron small-angle X-ray diffraction (SAXD) measurements illustrating the liquid crystalline structure of the SPC/Span^{®}80 mixtures.
Figure 3 shows synchrotron small-angle X-ray diffraction (SAXD) measurements illustrating the liquid crystalline structure of the DOPC/Span^{®}80 mixtures.
Figure 4 shows synchrotron small-angle X-ray diffraction (SAXD) measurements illustrating the liquid crystalline structure of the DOPE/Span^{®}80 mixtures.
Figure 5 shows X-ray diffraction patterns of fully hydrated SPC/Span^{®}80/VitEAc mixtures. (comparative example)
Figure 6 shows the in vitro release of leuprolide acetate from SPC/Span^{®}80 and comparative SPC/Span^{®}80/VitEAc and SPC/GDO formulations containing 2.1 wt% of LEU.
Figure 7 shows the in vitro release of octreotide from SPC/Span^{®}80 and comparative SPC/Span^{®}80/VitEAc and SPC/GDO formulations containing 2.3 wt% of OCT.

### Examples

### Materials

Soy phosphatidylcholine (SPC) -Lipoid S100 from Lipoid, Germany
Dioleoylphosphatidylcholine (DOPC) - from NOF, Japan
Dioleoylphosphatidylethanolamine (DOPE) - Lipoid PE 18:1/18:1 from Lipoid, Germany
Sorbitan monooleate (Span^{®}80) - from Sigma-Aldrich, Sweden
Vitamin E acetate (VitEAc) - from Sigma-Aldrich, Sweden
Glycerol dioleate (GDO) - Cithrol GDO from Croda, UK
Ethanol (EtOH) 99.5% Ph. Eur. - from Solveco, Sweden
Leuprolide acetate (LEU) - from PolyPeptide Labs., USA
Octreotide hydrochloride (OCT) - from PolyPeptide Labs., USA
Phosphate buffered saline (PBS) tablets - from Sigma-Aldrich, Sweden
Water for Injection (WFI) - from B. Braun, Germany
All other chemicals were of analytical grade purity

### Example 1.

### Liquid formulations comprising soy phosphatidylcholine and Span^{®}80

Precursor formulations containing different proportions of soy phosphatidylcholine (SPC), sorbitan monooleate (Span^{®}80) and ethanol (EtOH) as solvent were prepared. Appropriate amounts of SPC, Span^{®}80 and EtOH (3 g in total) were weighed in 6R injection glass vials. Sealed vials were then placed on a roller mixer at room temperature until mixed completely into clear homogeneous liquid solution (<24 hours). Sample compositions are given in Table 2.

**Table 2. Compositions of SPC/Span^{®}80/EtOH formulations.**

| Formulation No | SPC (wt%) | Span^{®}80 (wt%) | EtOH (wt%) | SPC/Span^{®}80 (weight ratio) |
|---|---|---|---|---|
| #1 | 63.00 | 27.00 | 10.00 | 70/30 |
| #2 | 54.00 | 36.00 | 10.00 | 60/40 |
| #3 | 49.50 | 40.50 | 10.00 | 55/45 |
| #4 | 45.00 | 45.00 | 10.00 | 50/50 |
| #5 | 40.50 | 49.50 | 10.00 | 45/55 |
| #6 | 36.00 | 54.00 | 10.00 | 40/60 |
| #7 | 31.50 | 58.50 | 10.00 | 35/65 |
| #8 | 27.00 | 63.00 | 10.00 | 30/70 |
| #9 | 22.50 | 67.50 | 10.00 | 25/75 |
| #10 | 18.00 | 72.00 | 10.00 | 20/80 |
| #11^{∗} | 13.50 | 76.50 | 10.00 | 15/85 |
| #12^{∗} | 9.00 | 81.00 | 10.00 | 10/90 |

| | | | | |
|---|---|---|---|---|
| ^{∗}comparative examples | | | | |

### Example 2.

### Liquid formulations comprising dioleoylphosphatidylcholine and Span^{®}80

Precursor formulations containing different proportions of dioleoylphosphatidylcholine (DOPC), sorbitan monooleate (Span^{®}80) and ethanol (EtOH) as solvent were prepared. Appropriate amounts of DOPC, Span^{®}80 and EtOH (3 g in total) were weighed in 6R injection glass vials. Sealed vials were then placed on a roller mixer at room temperature until mixed completely into clear homogeneous liquid solution (<24 hours). Sample compositions are given in Table 3.

**Table 3. Compositions of DOPC/Span^{®}8Q/EtOH formulations.**

| Formulation No | DOPC (wt%) | Span^{®}80 (wt%) | EtOH (wt%) | DOPC/Span^{®}80 (weight ratio) |
|---|---|---|---|---|
| #13 | 54.00 | 36.00 | 10.00 | 60/40 |
| #14 | 45.00 | 45.00 | 10.00 | 50/50 |
| #15 | 36.00 | 54.00 | 10.00 | 40/60 |

### Example 3.

### Liquid formulations comprising dioleoylphosphatidylethanolamine and Span^{®}80

Precursor formulations containing different proportions of dioleoylphosphatidylethanolamine (DOPE), sorbitan monooleate (Span^{®}80) and ethanol (EtOH) as solvent were prepared. Appropriate amounts of DOPE, Span^{®}80 and EtOH (3 g in total) were weighed in 6R injection glass vials. Sealed vials were then placed on a roller mixer at room temperature until mixed completely into clear homogeneous liquid solution (<24 hours). Sample compositions are given in Table 4.

**Table 4. Compositions of DOPE/Span^{®}80/EtOH formulations.**

| Formulation No | DOPE (wt%) | Span^{®}80 (wt%) | EtOH (wt%) | DOPE/Span^{®}80 (weight ratio) |
|---|---|---|---|---|
| #16 | 54.00 | 36.00 | 10.00 | 60/40 |
| #17 | 45.00 | 45.00 | 10.00 | 50/50 |
| #18 | 36.00 | 54.00 | 10.00 | 40/60 |

### Example 4. Liquid formulations comprising soy phosphatidylcholine, vitamin E acetate and Span^{®}80 (comparative example)

For comparison, formulations containing different proportions of soy phosphatidylcholine (SPC), sorbitan monooleate (Span^{®}80), ethanol (EtOH) as solvent and vitamin E acetate (VitEAc) as liquid crystalline "hardener" were prepared. Appropriate amounts of SPC, Span^{®}80, EtOH and VitEAc (3 g in total) were weighed in 6R injection glass vials. Sealed vials were then placed on a roller mixer at room temperature until mixed completely into clear homogeneous liquid solution (<24 hours). Sample compositions are given in Table 5.

**Table 5. Compositions of SPC/Span^{®}80/VitEAc/EtOH formulations.**

| Formulation No | SPC (wt%) | Span^{®}80 (wt%) | VitEAc (wt%) | EtOH (wt%) | SPC/(Span^{®}80+VitEAc) (weight ratio) |
|---|---|---|---|---|---|
| #19 | 63.00 | 18.00 | 9.00 | 10.00 | 70/30 |
| #20 | 54.00 | 27.00 | 9.00 | 10.00 | 60/40 |
| #21 | 45.00 | 36.00 | 9.00 | 10.00 | 50/50 |
| #22 | 36.00 | 45.00 | 9.00 | 10.00 | 40/60 |
| #23 | 27.00 | 54.00 | 9.00 | 10.00 | 30/70 |

### Example 5.

### Liquid formulations comprising soy phosphatidylcholine and glycerol dioleate (comparative example)

For comparison, formulations containing different proportions of soy phosphatidylcholine (SPC), glycerol dioleate (GDO) and ethanol (EtOH) as solvent were prepared. Appropriate amounts of SPC, GDO and EtOH (3 g in total) were weighed in 6R injection glass vials. Sealed vials were then placed on a roller mixer at room temperature until mixed completely into clear homogeneous liquid solution (<24 hours). Sample compositions are given in Table 6.

**Table 6. Compositions of SPC/GDO/EtOH formulations.**

| Formulation No | SPC (wt%) | GDO (wt%) | EtOH (wt%) | SPC/GDO (weight ratio) |
|---|---|---|---|---|
| #24 | 63.00 | 27.00 | 10.00 | 70/30 |
| #25 | 54.00 | 36.00 | 10.00 | 60/40 |
| #26 | 49.50 | 40.50 | 10.00 | 55/45 |
| #27 | 45.00 | 45.00 | 10.00 | 50/50 |
| #28 | 40.50 | 49.50 | 10.00 | 45/55 |
| #29 | 36.00 | 54.00 | 10.00 | 40/60 |
| #30 | 31.50 | 58.50 | 10.00 | 35/65 |
| #31 | 27.00 | 63.00 | 10.00 | 30/70 |

### Example 6.

### Viscosity of liquid formulations comprising phospholipid and Span^{®}80

Viscosity measurements were performed on formulations prepared in Examples 1-5. Measurements were performed using CAP 2000+ high torque viscometer (Brookfield, MA) equipped with CAP01 cone spindle at a share rate of 4000 s⁻¹ (rotation speed 300 rpm) at 25 °C. 75 µl of the formulation was placed between holding plate and cone spindle, equilibrated for 10 s and measured for 15 s.

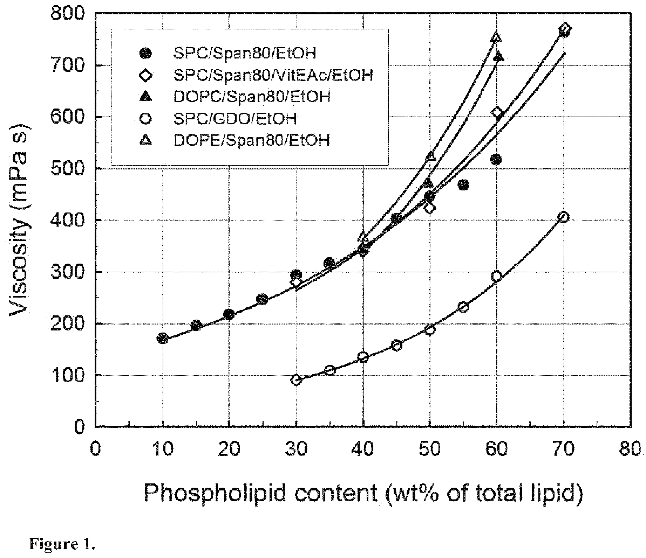

Figure 1 illustrates formulation viscosity against the proportion of phospholipid in respect to total lipid content. The general trend is that lower PC content in respect to total lipid results in a lower viscosity formulation. Formulations of the Invention illustrated in Figure 1 include SPC/Span^{®}80/EtOH, DOPC/Span^{®}80/EtOH and DOPE/Span^{®}80/EtOH. The viscosity of these Formulations can be as low as the comparative SPC/GDO/EtOH system, although a significantly lower PC content is required in the PC/Span^{®}80 system to achieve the same viscosity as in the PC/GDO system. The viscosity of the phospholipid/Span/EtOH system is practically not affected by the additional presence of VitEAc.

### Example 7.

### Liquid crystalline phase structures from phospholipid/Span^{®}80 mixtures in the presence of aqueous phase

200 mg of each of the formulation from Examples 1-5 was injected into 5 mL PBS solution in injection 10R glass vials using disposable 1 mL Luer-Lock syringes and 21G needles. Prepared samples were left to equilibrate for 1 week before further analysis.

The nanostructure of equilibrated liquid crystalline phases was studied using synchrotron small-angle X-ray diffraction (SAXD) measurements, which were performed at the I911-4beamline at MAX-lab (Lund University, Sweden), using a 1M PILATUS 2D detector containing a total of 981 × 1043 pixels. Samples were mounted between kapton windows in a steel sample holder at the sample to detector distance of 1917 mm. Diffractograms were recorded with a wavelength of 0.91 Å and the beam size of 0.25 × 0.25 mm (full width at the half-maximum) at the sample. Silver behenate calibrated sample-to-detector distance and detector positions were used. Temperature control within 0.1°C was achieved using computer controlled Julabo heating circulator F12-MC (Julabo Labortechnik GMBH, Seelbach, Germany). The experiments were performed successively at 25, 37, and 42 °C with a 60 s exposure time at each temperature and a wait of 10 minutes between temperature steps. The resulting CCD images were integrated and analyzed using the Fit2D software.

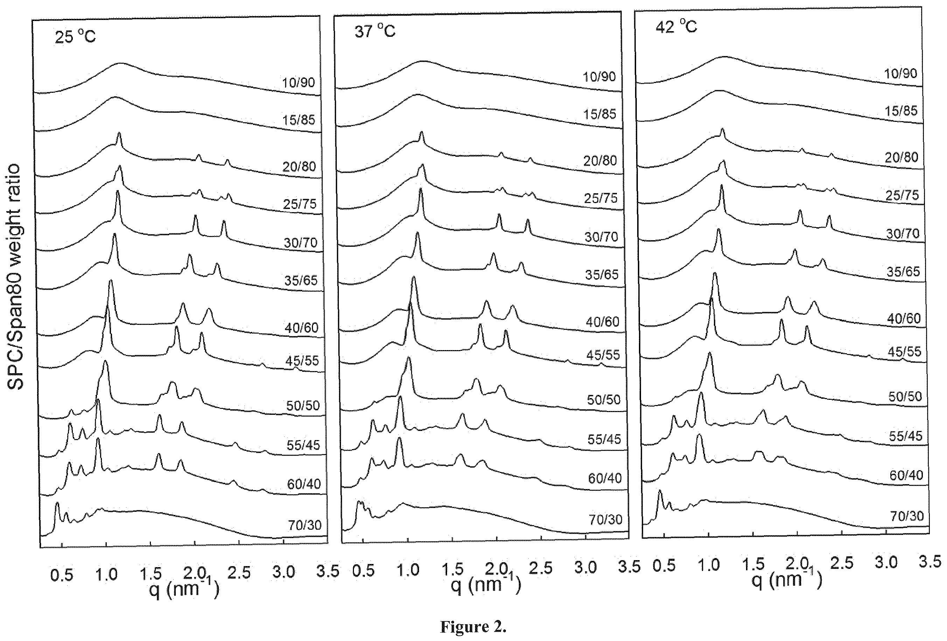

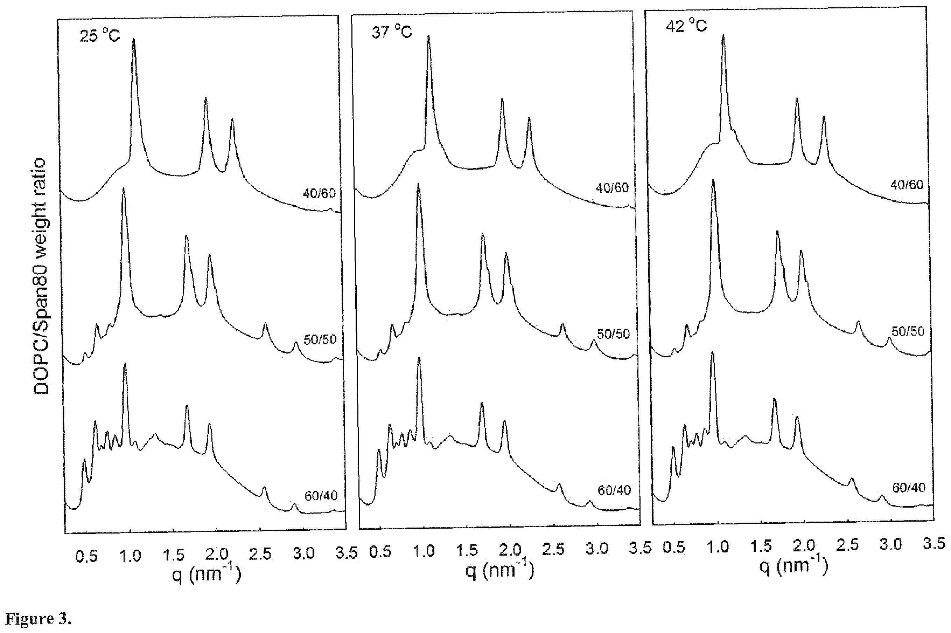

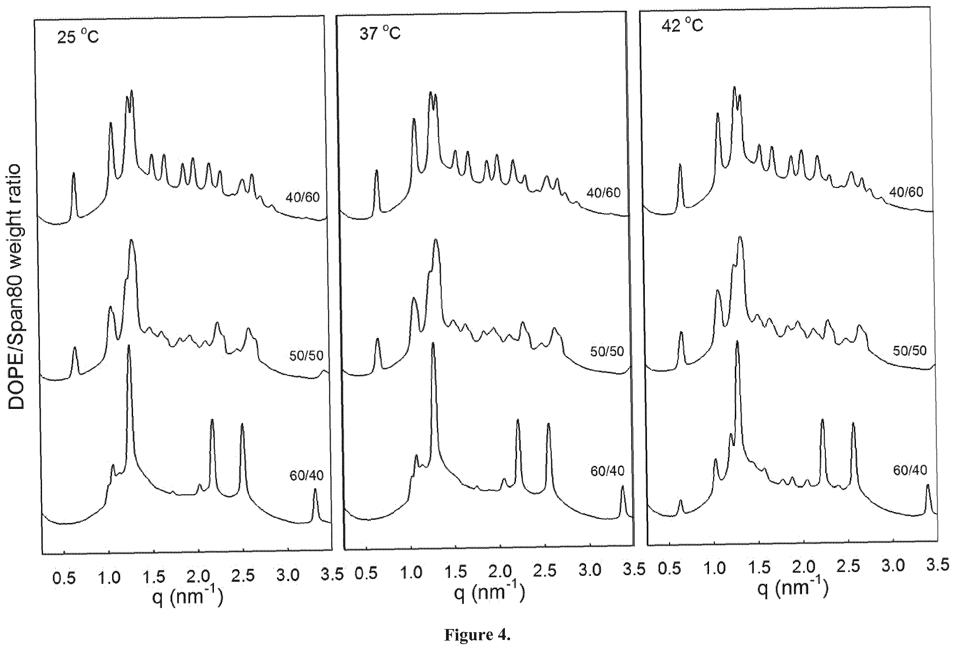

The obtained results for various lipid mixtures are summarized in Figures 2-5. The relative diffraction peak positions in Figure 2 indicate that the liquid crystalline structure of the SPC/Span^{®}80 mixtures changes from reversed bicontinuous cubic (V₂) at low Span^{®}80 content to reversed hexagonal (H₂) and then to reversed micellar phase (L₂) when the Span^{®}80 content is increased. Figure 3 shows that the liquid crystalline structure of the DOPC/Span^{®}80 changes from a mixture of reversed bicontinuous cubic (V₂) and reversed hexagonal (H₂) phase at DOPC/Span^{®}80 weight ratios of 60/40 and 50/50 to pure H₂ phase at DOPC/Span^{®}80 weight ratio of 40/60. The relative diffraction peak positions in Figure 4 indicate that the liquid crystalline structure of the DOPE/Span^{®}80 changes from a mixture of reversed micellar cubic (I₂, space group Fd3m) and reversed hexagonal (H₂) phase at DOPE/Span^{®}80 weight ratio of 60/40 to pure reversed micellar cubic (I₂, space group Fd3m) at DOPE/Span^{®}80 weight ratios of 50/50 and 40/60.

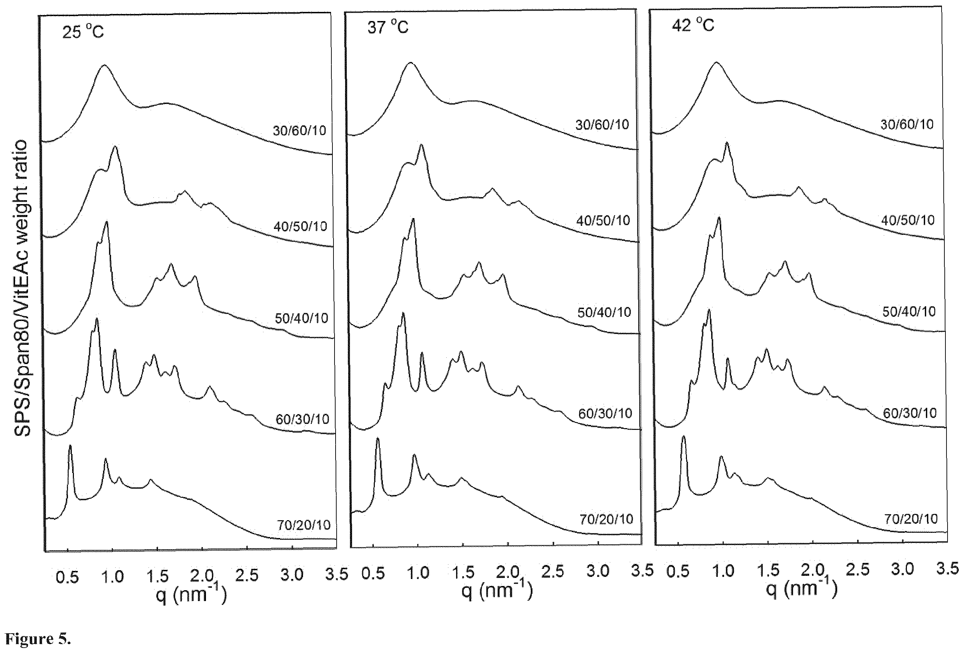

For comparison, Figure 5 shows X-ray diffraction patterns of fully hydrated SPC/Span^{®}80/VitEAc mixtures between weight ratios of 70/20/10 and 30/60/10 as indicated in the figure. The relative diffraction peak positions indicate that the liquid crystalline structure changes from reversed mixtures of bicontinuous cubic (V₂) and intermediate phase at low Span^{®}80 content to reversed reversed hexagonal (H₂) and then to reversed micellar phase (L₂) when the Span^{®}80 content is increased.

Overall, data presented in Figures 2-4 show a general trend of the non-lamellar phase formation in lipid mixtures comprising phospholipid and Span^{®}80: At high phospholipid content, bicontinuous structures are formed which with increasing Span^{®}80 proportion in the mixture first are transformed into reversed hexagonal (or reversed micellar cubic phase in the case of DOPE) and then into reversed micellar phase. When comparing with Figure 5, the data also show that the presence of 10 wt% (of total lipid content) of liquid crystal "hardener" (VitEAc) does not influence the type of the non-lamellar liquid crystalline structures or the observed phase transformation sequence observed without the VitEAc.

### Example 8.

### In vitro release of leuprolide acetate from phospholipid/Span^{®}80 mixtures in the presence of aqueous phase

To 0.95 g of each of the formulations #4, #6, #21, #22, #27 and #29 was added 29 mg of DMSO and 21 mg of leuprolide acetate (LEU) to get 2.1 wt% (or 2.0 wt% when corrected for peptide content and purity) of LEU in total. Assignment of the prepared samples (L1-L6) is given in Table 7.

**Table 7. Compositions of LEU containing formulations for in vitro release experiments.**

| Sample No | Formulation (g) | LEU (g) | DMSO (g) | Lipid weight ratio (wt%) |
|---|---|---|---|---|
| L1 | 0.95 | 0.021 | 0.029 | SPC/Span^{®}80 = 50/50 |
| L2 | 0.95 | 0.021 | 0.029 | SPC/Span^{®}80 = 40/60 |
| L3^{∗} | 0.95 | 0.021 | 0.029 | SPC/Span^{®}80/VitEAc = 50/40/10 |
| L4^{∗} | 0.95 | 0.021 | 0.029 | SPC/Span^{®}80/VitEAc = 40/50/10 |
| L5^{∗} | 0.95 | 0.021 | 0.029 | SPC/GDO = 50/50 |
| L6^{∗} | 0.95 | 0.021 | 0.029 | SPC/GDO = 40/60 |

| | | | | |
|---|---|---|---|---|
| ^{∗}comparative examples | | | | |

5 mL PBS solution was added into injection vials (6R), followed by slow addition (with the help of a 1 mL single-use Luer Lock syringe equipped with an 18G needle) of approximately 100 mg/vial of each sample (L1-L6) containing LEU (3 replicates/formulation). The vials were sealed and placed on a shaking table (150 rpm) at 37°C. Sampling from each vial (200 µl/sample) was carried out after 24 h, 48 h and 14 days of incubation, and the aliquots were transferred into polypropylene HPLC micro vials.

Determination of LEU in the samples from in vitro release experiments was carried out by HPLC-UV, against calibration standards of the LEU in PBS, prepared in the concentration range 0.2 - 100 µg/mL (covering approximately the release range 0.05 - 25% of the maximal theoretical amount of peptide to be released). The HPLC-UV conditions were: Analytical column: ACE Excel 2 C18, 20x2.1 mm; column temperature: 50 °C; Mobile phase A (MP A): 0.1% trifluoroacetic acid (TFA) in water; Mobile phase B (MP B): 0.1% TFA in acetonitrile: methanol: water (90:5:5 v/v); Flow rate: 0.6 mL/min; Gradient: t0.0: 10% MP B; t0.2: 10% MP B; t4.2: 100% MP B; t4.7: 100% MP B; t5.0: 10% MP B; t6.5: 10% MP B; Injection volume: 10 µL; Detection wavelength: 220 nm.

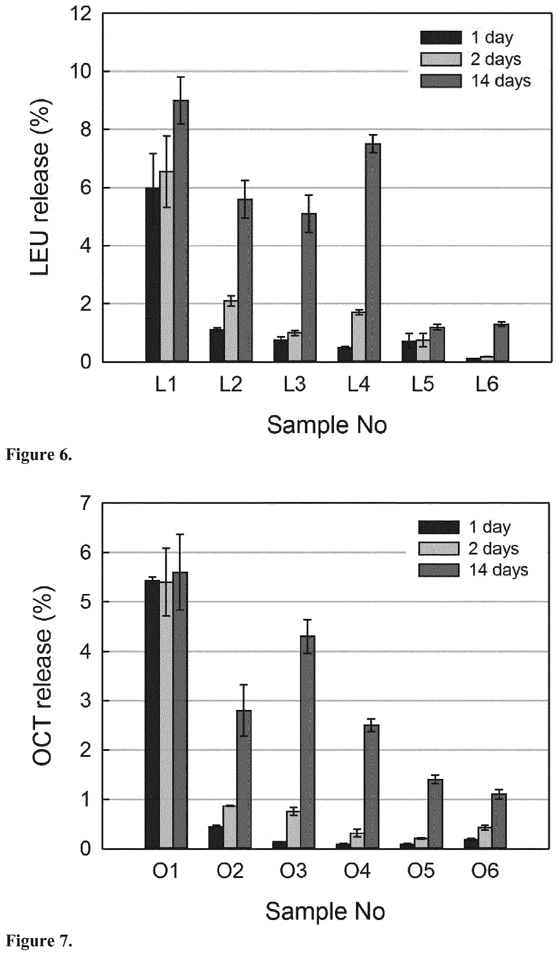

Figure 6 illustrates the in vitro release of LEU from SPC/Span^{®}80 and comparative SPC/Span^{®}80/VitEAc and SPC/GDO formulations containing 2.1 wt% of LEU. The data clearly show a dramatic reduction in the burst release seen after 1 and 2 days when moving from a SPC/Span^{®}80 weight ratio of 50/50 (L1) to ratio of 40/60 (L2) which may be related to the different non-lamellar nanostructure (H₂ phase) in the latter case. After 14 days, a clear difference in released amount of LEU between the formulations is still observed. Importantly, the addition of the liquid crystal "hardener" VitEAc (samples L3 and L4) did not slow down the release of LEU. After 14 days the released amount of LEU from samples L3 and L4 was practically the same as in the case of SPC/Span^{®}80 formulation prepared at lipid weight ratio of 40/60 (L2). The comparative SPC/GDO formulations (L5, L6) showed the slowest in vitro LEU release, both initially and up to 14 days.

### Example 9.

### In vitro release of octreotide hydrochloride from phospholipid/Span^{®}80 mixtures in the presence of aqueous phase

To 0.977 g of each of the formulations #4, #6, #21, #22, #27 and #29 was added 23 mg of octreotide hydrochloride (OCT) to get 2.3 wt% (or 2.0 wt% when corrected for peptide content and purity) of OCT in total. Assignment of the prepared samples (O1-O6) is given in Table 8.

**Table 8. Compositions of OCT containing formulations for in vitro release experiments.**

| Sample No | Formulation (g) | OCT (g) | Lipid weight ratio (wt%) |
|---|---|---|---|
| O1 | 0.977 | 0.023 | SPC/Span^{®}80 = 50/50 |
| O2 | 0.977 | 0.023 | SPC/Span^{®}80 = 40/60 |
| O3^{∗} | 0.977 | 0.023 | SPC/Span^{®}80/VitEAc = 50/40/10 |
| O4^{∗} | 0.977 | 0.023 | SPC/Span^{®}80/VitEAc = 40/50/10 |
| O5^{∗} | 0.977 | 0.023 | SPC/GDO = 50/50 |
| O6^{∗} | 0.977 | 0.023 | SPC/GDO = 40/60 |

| | | | |
|---|---|---|---|
| ^{∗}comparative examples | | | |

In vitro release experiments were further carried out as in Example 8 (the same HPLC assay but with calibration standards of OCT in PBS).

Figure 7 illustrates the in vitro release of OCT from SPC/Span^{®}80 and comparative SPC/Span^{®}80/VitEAc and SPC/GDO formulations containing 2.3 wt% of OCT. Overall, the obtained data are very similar to that given in Example 8. Here also a dramatic reduction in the burst release seen after 1 and 2 days when moving from a SPC/Span^{®}80 ratio of 50/50 (O1) to ratio of 40/60 (O2) is observed which may be related to the different non-lamellar nanostructure (H₂ phase) in the latter case. After 14 days, a clear difference in released amount of OCT between the formulations is still observed. Importantly, the addition of the liquid crystal "hardener" VitEAc (samples O3 and O4) did not markedly change the release profile of OCT. After 14 days the released amount of OCT from samples O3 and O4 was practically the same or higher compared with the SPC/Span^{®}80 formulation prepared at lipid weight ratio of 40/60 (O2). The comparative SPC/GDO formulations (O5, O6) showed the slowest in vitro OCT release, both initially and up to 14 days.

## Claims

1. A pre-formulation comprising a low viscosity, non-liquid crystalline, mixture of:
i) at least one sorbitan ester;
ii) at least one phospholipid;
iii) at least one biocompatible, oxygen containing, low viscosity organic solvent;
wherein component i) comprises at least one fatty acid ester of a sorbitan comprising a sorbitan head group and one to three fatty acyl tail groups;
wherein the weight ratio of i) : ii) is in the range of 30:70 to 80:20;
wherein the pre-formulation forms, or is capable of forming, at least one non-lamellar liquid crystalline phase structure upon contact with an aqueous fluid; and wherein the pre-formulation does not further comprise any of; triglycerides, retinyl palmitate, benzyl benzoate, cholesterol, ubiquinone, tocopherols or mixtures thereof; or the levels of triglycerides, retinyl palmitate, benzyl benzoate, ubiquinone, tocopherols, or cholesterol in the pre-formulation are all below 1,000 ppm by weight relative to the pre-formulation as a whole.

2. A pre-formulation according to claim 1 which does not comprise any component free of an ionizable group, having a hydrophobic moiety of 15 to 40 carbon atoms with a triacyl group or a carbon ring structure.

3. A pre- formulation according to claim 1 or claim 2 wherein the fatty acid ester of a sorbitan comprising a sorbitan head group and one to three fatty acyl tail groups is a fatty acid sorbitan di-ester comprising a sorbitan head group and two fatty acyl tail groups.

4. A pre-formulation according to any preceding claim wherein each fatty acyl tail group is independently selected from caproic, caprylic, capric, lauric, myristic, palmitic, phytanic, palmitolic, stearic, iso-stearic, oleic, elaidic, linoleic, linolenic, arachidonic, behenic or lignoceric acids, preferably palmitic, stearic, oleic and linoleic acids, particularly oleic acid.

5. A pre-formulation according to any preceding claim wherein component ii) is at least one phosphatidyl choline or at least one phosphatidyl ethanolamine or mixtures thereof.

6. A pre-formulation according to any preceding claim wherein the weight ratio of i) : ii) is in the range of 45:55 to 75:25.

7. A pre-formulation according to any preceding claim wherein component i) comprises at least 30% fatty acid di-esters of sorbitan and component ii) is soy PC, wherein the weight ratio of i) : ii) is 45:55 to 75:25, preferably 50:50 to 75:25, more preferably 55:45 to 70:30.

8. A pre-formulation according to any preceding claim further comprising at least one active agent.

9. A pre-formulation as claimed in claim 8 wherein said active agent is selected from opioid agonists, opioid antagonists, GnRH agonists, (buserelin, deslorelin, goserelin, leuprorelin/leuprolide, naferelin and triptorelin), GnRH antagonists(cetrorelix, ganirelix, abarelix, degarelix), somatostatins (SST-14 and SST-28) and somatostatin receptor (SSTR) agonists, e.g. octreotide, lanreotide, vapreotide, pasireotide, glucagon-like peptide 1 (GLP-1) receptor agonists (GLP-1(7-37), GLP-1(7-36)amide), liraglutide, exenatide, and lixisenatide (AVE0010)), and glucagon-like peptide 2 agonists (e.g. ZP1846), and mixtures thereof.

10. A pre-formulation according to claim 8 wherein the active agent is an opioid agonist selected from buprenorphine, fentanyl, sufentanil, remifentanil, oxymorphone, dimorphone, dihydroetorphine or diacetylmorphine; or wherein the active agent is an opioid antagonist selected from naloxone, nalmefene or naltrexone.

11. A pre-formulation according to claim 8 wherein said active agent is a cyclic peptide of 30 or fewer amino acids, preferably 15 or fewer.

12. A pre-formulation according to claim 1 wherein the levels of triglycerides, retinyl palmitate, benzyl benzoate, ubiquinone, tocopherols or cholesterol in the pre-formulation are below 1,000 ppm by weight relative to the pre-formulation as a whole.

13. A pre-formulation as claimed in any of claims 1 to 12 for use as a medicament.

14. A pre-filled administration device containing a pre-formulation as claimed in any of claims 1 to 12, said device being optionally equipped with an injection aid, such as an auto-injector.

15. A kit comprising an administration device as claimed in claim 14.

## Patentansprüche

1. Vorformulierung, umfassend eine niederviskose, nicht flüssigkristalline Mischung von:
i) mindestens einem Sorbitanester;
ii) mindestens einem Phospholipid;
iii) mindestens einem biokompatiblen, sauerstoffhaltigen, niederviskosen organischen Lösungsmittel;
wobei Komponente i) mindestens einen Fettsäureester eines Sorbitans mit einer Sorbitan-Kopfgruppe und einer bis drei Fettacyl-Schwanzgruppen umfasst;
wobei das Gewichtsverhältnis von i) : ii) im Bereich von 30:70 bis 80:20 liegt;
wobei die Vorformulierung bei Kontakt mit einem wässrigen Fluid mindestens eine nichtlamellare flüssigkristalline Phasenstruktur bildet oder bilden kann und
wobei die Vorformulierung nicht weiterhin Triglyceride, Retinylpalmitat, Benzylbenzoat, Cholesterin, Ubichinon, Tocopherole oder Mischungen davon umfasst oder die Gehalte von Triglyceriden, Retinylpalmitat, Benzylbenzoat, Ubichinon, Tocopherolen oder Cholesterin alle unter 1000 Gew.-ppm, bezogen auf die Vorformulierung als Ganzes, liegen.

2. Vorformulierung nach Anspruch 1, die keine von einer ionisierbaren Gruppe freie Komponente mit einer hydrophoben Gruppierung von 15 bis 40 Kohlenstoffatomen mit einer Triacylgruppe oder einer Kohlenstoffringstruktur umfasst.

3. Vorformulierung nach Anspruch 1 oder Anspruch 2, wobei es sich bei dem Fettsäureester eines Sorbitans mit einer Sorbitan-Kopfgruppe und einer bis drei Fettacyl-Schwanzgruppen um einen Fettsäuresorbitandiester mit einer Sorbitan-Kopfgruppe und zwei Fettacyl-Schwanzgruppen handelt.

4. Vorformulierung nach einem der vorhergehenden Ansprüche, wobei jede Fettacyl-Schwanzgruppe unabhängig aus Capronsäure, Caprylsäure, Caprinsäure, Laurinsäure, Myristinsäure, Palmitinsäure, Phytansäure, Palmitoleinsäure, Stearinsäure, Isostearinsäure, Ölsäure, Elaidinsäure, Linolsäure, Linolensäure, Arachidonsäure, Behensäure oder Lignocerinsäure, vorzugsweise Palmitinsäure, Stearinsäure, Ölsäure und Linolsäure, insbesondere Ölsäure, ausgewählt ist.

5. Vorformulierung nach einem der vorhergehenden Ansprüche, wobei es sich bei Komponente ii) um mindestens ein Phosphatidylcholin oder mindestens ein Phosphatidylethanolamin oder Mischungen davon handelt.

6. Vorformulierung nach einem der vorhergehenden Ansprüche, wobei das Gewichtsverhältnis von i) : ii) im Bereich von 45:55 bis 75:25 liegt.

7. Vorformulierung nach einem der vorhergehenden Ansprüche, wobei Komponente i) mindestens 30 % Fettsäurediester von Sorbitan umfasst und es sich bei Komponente ii) um Soja-PC handelt, wobei das Gewichtsverhältnis von i): ii) 45:55 bis 75:25, vorzugsweise 50:50 bis 75:25, weiter bevorzugt 55:45 bis 70:30, beträgt.

8. Vorformulierung nach einem der vorhergehenden Ansprüche, die ferner mindestens einen Wirkstoff enthält.

9. Vorformulierung nach Anspruch 8, wobei der Wirkstoff aus Opioid-Agonisten, Opioid-Antagonisten, GnRH-Agonisten (Buserelin, Deslorelin, Goserelin, Leuprorelin/Leuprolid, Naferelin und Triptorelin), GnRH-Antagonisten (Cetrorelix, Ganirelix, Abarelix, Degarelix), Somatostatinen (SST-14 und das SST-28) und Somatostatin-Rezeptor(SSTR)-Agonisten, z. B. Octreotid, Lanreotid, Vapreotid, Pasireotid, Glucagon-like-Peptide-1(GLP-1)-Rezeptoragonisten) (GLP-1(7-37), GLP-1(7-36)-Amid), Liraglutid, Exenatid und Lixisenatid (AVE0010)) und Glucagon-like-Peptid-2-Agonisten (z. B. ZP1846) und Mischungen davon ausgewählt ist.

10. Vorformulierung nach Anspruch 8, wobei es sich bei dem Wirkstoff um einen Opioid-Agonisten handelt, der aus Buprenorphin, Fentanyl, Sufentanil, Remifentanil, Oxymorphon, Dimorphon, Dihydroetorphin oder Diacetylmorphin ausgewählt ist; oder wobei es sich bei dem Wirkstoff um einen Opioid-Antagonisten handelt, der aus Naloxon, Nalmefen oder Naltrexon ausgewählt ist.

11. Vorformulierung nach Anspruch 8, wobei es sich bei dem Wirkstoff um ein cyclisches Peptid aus 30 oder weniger und vorzugsweise 15 oder weniger Aminosäuren handelt.

12. Vorformulierung nach Anspruch 1, wobei die Gehalte von Triglyceriden, Retinylpalmitat, Benzylbenzoat, Ubichinon, Tocopherolen oder Cholesterin in der Vorformulierung unter 1000 Gew.-ppm, bezogen auf die Vorformulierung als Ganzes, liegen.

13. Vorformulierung nach einem der Ansprüche 1 bis 12 zur Verwendung als Medikament.

14. Vorgefüllte Verabreichungsvorrichtung, enthaltend eine Vorformulierung nach einem der Ansprüche 1 bis 12, wobei die Vorrichtung gegebenenfalls mit einer Injektionshilfe, wie einem Autoinjektor, ausgestattet ist.

15. Kit, umfassend eine Verabreichungsvorrichtung nach Anspruch 14.

## Revendications

1. Pré-formulation comprenant un mélange non de type cristallin liquide, à faible viscosité composé de :
i) au moins un ester de sorbitane ;
ii) au moins un phospholipide ;
iii) au moins un solvant organique biocompatible, contenant de l'oxygène, à faible viscosité ;
le composant i) comprenant au moins un ester d'acide gras d'un sorbitane comprenant un groupe de tête de type sorbitane et un à trois groupes de queue de type acyle gras ;
le rapport en poids de i) : ii) étant dans la plage de 30 : 70 à 80 : 20 ;
la pré-formulation formant, ou étant capable de former, au moins une structure de phase cristalline liquide non lamellaire lors d'un contact avec un fluide aqueux ; et
la pré-formulation ne comprenant en outre aucun parmi : des triglycérides, le palmitate de rétinyle, le benzoate de benzyle, le cholestérol, l'ubiquinone, des tocophérols et des mélanges correspondants ;
ou les taux de triglycérides, de palmitate de rétinyle, de benzoate de benzyle, d'ubiquinone, de tocophérols ou de cholestérol dans la pré-formulation étant tous inférieurs à 1 000 ppm en poids par rapport à la pré-formulation dans son ensemble.

2. Pré-formulation selon la revendication 1 qui ne comprend pas un quelconque composant exempt d'un groupe ionisable, possédant un fragment hydrophobe de 15 à 40 atomes de carbone avec un groupe triacyle ou une structure cyclique carbonée.

3. Pré-formulation selon la revendication 1 ou la revendication 2, l'ester d'acide gras d'un sorbitane comprenant un groupe de tête de type sorbitane et un à trois groupes de queue de type acyle gras étant un di-ester de sorbitane d'acide gras comprenant un groupe de tête de type sorbitane et deux groupes de queue de type acyle gras.

4. Pré-formulation selon une quelconque revendication précédente, chaque groupe de queue de type acyle gras étant indépendamment choisi parmi les acides caproïque, caprylique, caprique, laurique, myristique, palmitique, phytanique, palmitoléique, stéarique, iso-stéarique, oléique, élaïdique, linoléique, linolénique, arachidonique, béhénylique et lignocérique, préférablement les acides palmitique, stéarique, oléique et linoléique, particulièrement l'acide oléique.

5. Pré-formulation selon une quelconque revendication précédente, le composant ii) étant au moins une phosphatidylcholine ou au moins une phosphatidyléthanolamine ou des mélanges correspondants.

6. Pré-formulation selon une quelconque revendication précédente, le rapport en poids de i) : ii) étant dans la plage de 45 : 55 à 75 : 25.

7. Pré-formulation selon une quelconque revendication précédente, le composant i) comprenant au moins 30 % de di-esters d'acides gras de sorbitane et le composant ii) étant de la PC de soja, le rapport en poids de i) : ii) étant de 45 : 55 à 75 : 25, préférablement de 50 : 50 à 75 : 25, plus préférablement de 55 : 45 à 70 : 30.

8. Pré-formulation selon une quelconque revendication précédente comprenant en outre au moins un agent actif.

9. Pré-formulation selon la revendication 8, ledit agent actif étant choisi parmi des agonistes opioïdes, des antagonistes opioïdes, des agonistes de GnRH, (buséréline, desloréline, goséréline, leuproréline/leuprolide, naféréline et triptoréline), des antagonistes de GnRH (cétrorelix, ganirélix, abarélix, dégarélix), des somatostatines (SST-14 et SST-28) et des agonistes du récepteur de somatostatine (SSTR), par ex. octréotide, lanréotide, vapréotide, pasiréotide, des agonistes du récepteur de glucagon-like peptide 1 (GLP-1) (GLP-1(7-37), GLP-1(7-36)amide), liraglutide, exénatide, et lixisénatide (AVE0010), et des agonistes du glucagon-like peptide 2 (par ex. ZP1846), et des mélanges correspondants.

10. Pré-formulation selon la revendication 8, l'agent actif étant un agoniste opioïde choisi parmi buprénorphine, fentanyl, sufentanil, rémifentanil, oxymorphone, dimorphone, dihydroétorphine ou diacétylmorphine ; ou l'agent actif étant un antagoniste opioïde choisi parmi naloxone, nalméfène et naltrexone.

11. Pré-formulation selon la revendication 8, ledit agent actif étant un peptide cyclique de 30 acides aminés ou moins, préférablement de 15 ou moins.

12. Pré-formulation selon la revendication 1, les taux de triglycérides, de palmitate de rétinyle, de benzoate de benzyle, d'ubiquinone, de tocophérols ou de cholestérol dans la pré-formulation étant inférieurs à 1 000 ppm en poids par rapport à la pré-formulation dans son ensemble.

13. Pré-formulation selon l'une quelconque des revendications 1 à 12 pour une utilisation en tant que médicament.

14. Dispositif d'administration pré-rempli contenant une pré-formulation selon l'une quelconque des revendications 1 à 12, ledit dispositif étant éventuellement pourvu d'un élément d'assistance d'injection, tel qu'un auto-injecteur.

15. Kit comprenant un dispositif d'administration selon la revendication 14.
